(19) Europäisches Patentamt
European Patent Office
Office européen des brevets

(11) **EP 1 463 491 B1**

(12) **EUROPEAN PATENT SPECIFICATION**

(45) Date of publication and mention
of the grant of the patent:
**12.09.2012 Bulletin 2012/37**

(21) Application number: **02782151.1**

(22) Date of filing: **11.10.2002**

(51) Int Cl.:
*A61K 9/70* (2006.01)      *A61K 9/00* (2006.01)

(86) International application number:
**PCT/US2002/032575**

(87) International publication number:
**WO 2003/030882 (17.04.2003 Gazette 2003/16)**

(54) **THIN FILM WITH NON-SELF-AGGREGATING UNIFORM HETEROGENEITY, PROCESS FOR THER PRODUCTION AND DRUG DELIVERY SYSTEMS MADE THEREFROM**

DÜNNER FILM OHNE SELBTAGGREGATION MIT GLEICHMÄSSIGER HETEROGENITÄT, VERFAHREN ZU DEREN HERSTELLUNG, SOWIE DARAUS HERGESTELLTE ARZNEISTOFFABGABESYSTEME

FILM MINCE POURVU D'UNE HETEROGENEITE UNIFORME NON AUTOAGGLOMERANTE, SON PROCEDE D'ELABORATION ET SYSTEMES D'ADMINISTRATION DE MEDICAMENTS AINSI PRODUITS

(84) Designated Contracting States:
**AT BE BG CH CY CZ DE DK EE ES FI FR GB GR IE IT LI LU MC NL PT SE SK TR**

(30) Priority: **12.10.2001 US 328868 P**
**14.02.2002 US 74272**
**07.06.2002 US 386937 P**

(43) Date of publication of application:
**06.10.2004 Bulletin 2004/41**

(60) Divisional application:
**10182204.7 / 2 351 557**

(73) Proprietor: **MonoSolRX, LLC**
**Portage IN 46368 (US)**

(72) Inventors:
• **YANG, Robert, K.**
**Flushing, NY 11355 (US)**
• **FUISZ, Richard, C.**
**McLean, VA 22101 (US)**
• **MYERS, Gary, L.**
**Kingsport, TN 37660 (US)**
• **FUISZ, Joseph, M.**
**McLean, VA 22101 (US)**

(74) Representative: **Zeestraten, Albertus W. J. et al**
**Exter Polak & Charlouis B.V. (EP&C)**
**P.O. Box 3241**
**2280 GE Rijswijk (NL)**

(56) References cited:
EP-A- 0 514 691      EP-A- 1 110 546
WO-A-01/91721        US-A- 4 136 145
US-A- 4 631 837      US-A- 4 849 246
US-A- 4 925 670      US-A- 5 393 528
US-A- 5 629 003      US-B1- 6 284 264

Description

## FIELD OF THE INVENTION

The invention relates to rapidly dissolving films and methods of their preparation. The films may also contain an active ingredient that is evenly distributed throughout the film. The even or uniform distribution is achieved by controlling one or more parameters, and particularly the elimination of air pockets prior to and during film formation and the use of a drying process that reduces aggregation or conglomeration of the components in the film as it forms into a solid structure.

## BACKGROUND OF THE RELATED TECHNOLOGY

Active ingredients, such as drugs or pharmaceuticals, may be prepared in a tablet form to allow for accurate and consistent dosing. However, this form of preparing and dispensing medications has many disadvantages including that a large proportion of adjuvants that must be added to obtain a size able to be handled, that a larger medication form requires additional storage space, and that dispensing includes counting the tablets which has a tendency for inaccuracy. In addition, many persons, estimated to be as much as 28% of the population, have difficulty swallowing tablets. While tablets may be broken into smaller pieces or even crushed as a means of overcoming swallowing difficulties, this is not a suitable solution for many tablet or pill forms. For example, crushing or destroying the tablet or pill form to facilitate ingestion, alone or in admixture with food, may also destroy the controlled release properties.

As an alternative to tablets and pills, films may be used to carry active ingredients such as drugs, pharmaceuticals, and the like. However, historically films and the process of making drug delivery systems therefrom have suffered from a number of unfavorable characteristics that have not allowed them to be used in practice.

Films that incorporate a pharmaceutically active ingredient are disclosed in expired U.S. Patent No. 4,136,145 to Fuchs, et al. ("Fuchs"). These films may be formed into a sheet, dried and then cut into individual doses. The Fuchs disclosure alleges the fabrication of a uniform film, which includes the combination of water-soluble polymers, surfactants, flavors, sweeteners, plasticizers and drugs. These allegedly flexible films are disclosed as being useful for oral, topical or enteral use. Examples of specific uses disclosed by Fuchs include application of the films to mucosal membrane areas of the body, including the mouth, rectal, vaginal, nasal and ear areas.

Examination of films made in accordance with the process disclosed in Fuchs, however, reveals that such films suffer from the aggregation or conglomeration of particles, i.e., self-aggregation, making them inherently non-uniform. This result can be attributed to Fuchs' process parameters, which although not disclosed likely include the use of relatively long drying times, thereby facilitating intermolecular attractive forces, convection forces, air flow and the like to form such agglomeration.

The formation of agglomerates randomly distributes the film components and any active present as well. When large dosages are involved, a small change in the dimensions of the film would lead to a large difference in the amount of active per film. If such films were to include low dosages of active, it is possible that portions of the film may be substantially devoid of any active. Since sheets of film are usually cut into unit doses, certain doses may therefore be devoid of or contain an insufficient amount of active for the recommended treatment. Failure to achieve a high degree of accuracy with respect to the amount of active ingredient in the cut film can be harmful to the patient. For this reason, dosage forms formed by processes such as Fuchs, would not likely meet the stringent standards of governmental or regulatory agencies, such as the U.S. Federal Drug Administration ("FDA"), relating to the variation of active in dosage forms. Currently, as required by various world regulatory authorities, dosage forms may not vary more than 10% in the amount of active present. When applied to dosage units based on films, this virtually mandates that uniformity in the film be present.

The problems of self-aggregation leading to non-uniformity of a film were addressed in U.S. Patent No. 4,849,246 to Schmidt ("Schmidt"). Schmidt specifically pointed out that the methods disclosed by Fuchs did not provide a uniform film and recognized that that the creation of a non-uniform film necessarily prevents accurate dosing, which as discussed above is especially important in the pharmaceutical area. Schmidt abandoned the idea that a mono-layer film, such as described by Fuchs, may provide an accurate dosage form and instead attempted to solve this problem by forming a multi-layered film. Moreover, his process is a multi-step process that adds expense and complexity and is not practical for commercial use.

Other U.S. Patents directly addressed the problems of particle self-aggregation and non-uniformity inherent in conventional film forming techniques. In one attempt to overcome non-uniformity, U.S. Patent 5,629,003 to Horstmann et al. and U.S. Patent 5,948,430 to Zerbe et al. incorporated additional ingredients, i.e. gel formers and polyhydric alcohols respectively, to increase the viscosity of the film prior to drying in an effort to reduce aggregation of the components in the film. These methods have the disadvantage of requiring additional components, which translates to additional cost and manufacturing steps. Furthermore, both methods employ the use the conventional time-consuming drying methods such as a high-temperature air-bath using a drying oven, drying tunnel, vacuum drier, or other such drying equipment. The long length of drying time aids in promoting the aggregation of the active and other adjuvant, notwithstanding the use of viscosity modifiers. Such processes also run the risk of exposing the active, i.e., a drug, or vitamin C, or other components to prolonged exposure to moisture and elevated temperatures, which may render it ineffective or even harmful.

In addition to the concerns associated with degradation of an active during extended exposure to moisture, the conventional drying methods themselves are unable to provide uniform films. The length of heat exposure during conventional processing, often referred to as the "heat history", and the manner in which such heat is applied, have a direct effect on the formation and morphology of the resultant film product. Uniformity is particularly difficult to achieve via conventional drying methods where a relatively thicker film, which is well-suited for the incorporation of a drug active, is desired. Thicker uniform films are more difficult to achieve because the surfaces of the film and the inner portions of the film do not experience the same external conditions simultaneously during drying. Thus, observation of relatively thick films made from such conventional processing shows a non-uniform structure caused by convection and intermolecular forces and requires greater than 10% moisture to remain flexible. The amount of free moisture can often interfere over time with the drug leading to potency issues and therefore inconsistency in the final product.

WO 01/91,721 discloses film-forming compositions that can comprise, on a dry solid basis, 25 to 75 % by weight of certain starch derivatives having a dextrose equivalent less than about one, 25 to 75 % by weight plasticizer, and 0.1 to 15 % by weight of hydrocolloid gum. The film is formed by means of conventional drying methods.

US 6,284,264 discloses a composition containing therapeutic agents and/or breath freshening agents for use in the oral cavity. The carrier comprises water-soluble polymers in combination with certain ingredients and provides a therapeutic and/or cosmetic effect. The film is coated and dried utilizing existing coating technology.

In US 5,393,528 a dissolvable element containing an agent material is used for local administration of the agent material in an internal body area. The dissolvable element is made of dissolvable polymer material. As a contraceptive, the dissolvable element is preferably a film made of polyvinyl alcohol, polyethylene oxide, hydroxypropyl methyl cellulose and/or carboxymethyl cellulose. The dissolvable element may be formed as a laminate of different film layers for compound release properties. The film is dried by passing the composition through a drying oven.

EP 1,110,546 discloses a method of preparing a water soluble film, which method comprises (a) preparing a solution comprising a film former, a water soluble plasticizer, a pharmaceutically active agent, and a solvent; (b) drying the solution at a temperature of from about 50 to about 100 °C to form a film; and (c) curing the film at a temperature of from about 15 to about 60 ° C and at a relative humidity of at least about 30%. Preferably, the drying step is performed in two stages, e.g. in a drying tunnel.

EP 514,691 describes a type I collagen gel with an $H_2O$ content not exceeding 20% by weight, in the form of a sheet of thickness between 0.02 and 2 mm. The preparation thereof includes a drying step which is effected in a nitrogen stream under closed cycle.

US 4,925,670 discloses a dosage form for drug active agents, reagents or other active agents comprising a film-like carrier material having an active agent-containing coating one side thereof. The carrier material is in the form of a release paper, film or foil, and the active agent-containing coating is removed in a dosewise manner from the carrier material after subdivision into dosage units. When applying several layers, the layers are successively applied with each coating optionally directed to a drying station. This can comprise a thermostatically controlled pair of rollers and a drying tunnel.

Conventional drying methods generally include the use of forced hot air using a drying oven, drying tunnel, and the like. The difficulty in achieving a uniform film is directly related to the rheological properties and the process of water evaporation in the film-forming composition. When the surface of an aqueous polymer solution is contacted with a high temperature air current, such as a film-forming composition passing through a hot air oven, the surface water is immediately evaporated forming a polymer film or skin on the surface. This seals the remainder of the aqueous film-forming composition beneath the surface, forming a barrier through which the remaining water must force itself as it is evaporated in order to achieve a dried film. As the temperature outside the film continues to increase, water vapor pressure builds up under the surface of the film, stretching the surface of the film, and ultimately ripping the film surface open allowing the water vapor to escape. As soon as the water vapor has escaped, the polymer film surface reforms, and this process is repeated, until the film is completely dried. The result of the repeated destruction and reformation of the film surface is observed as a "ripple effect" which produces an uneven, and therefore non-uniform film. Frequently, depending on the polymer, a surface will seal so tightly that the remaining water is difficult to remove, leading to very long drying times, higher temperatures, and higher energy costs.

Other factors, such as mixing techniques, also play a role in the manufacture of a pharmaceutical film suitable for commercialization and regulatory approval. Air can be trapped in the composition during the mixing process or later during the film making process, which can leave voids in the film product as the moisture evaporates during the drying stage. The film frequently collapse around the voids resulting in an uneven film surface and therefore, non-uniformity of the final film product. Uniformity is still affected even if the voids in the film caused by air bubbles do not collapse. This situation also provides a non-uniform film in that the spaces, which are not uniformly distributed, are occupying area that would otherwise be occupied by the film composition. None of the above-mentioned patents either addresses or proposes a solution to the problems caused by air that has been introduced to the film.

Therefore, there is a need for methods and compositions for film products, which use a minimal number of materials or components, and which provide a substantially non-self-aggregating uniform heterogeneity throughout the area of the films. Desirably, such films are produced through a selection of a polymer or combination of polymers that will provide a desired viscosity, a film-forming process such as reverse roll coating, and a controlled, and desirably rapid, drying process which serves to maintain the uniform distribution of non-self-aggregated components without the necessary addition of gel formers or polyhydric alcohols and the like which appear to be required in the products and for the processes of prior patents, such as the aforementioned Horstmann and Zerbe patents. Desirably, the films will also incorporate compositions and methods of manufacture that substantially reduce or eliminate air in the film, thereby promoting uniformity in the final film product.

## SUMMARY OF THE INVENTION

In the present invention, there is provided a film product according to claim 1 and a method of forming the same according to claim 25, film product which can be divided into equally sized dosage units having substantially equal amounts of each compositional component present according to claim 31. This advantage is particularly useful because it permits large area films to be initially formed, and subsequently cut into individual dosage units without concern for whether each unit is compositionally equal. For example, the films of the present invention have particular applicability as pharmaceutical dosage delivery systems because each dosage unit, e.g., each individual dosage film unit, will contain the proper amount of drug. Pharmaceutical film dosage forms to date have not been marketed largely due to the inability to achieve this result.

In a further aspect of the present invention, there is provided a film product that is formed by combining a water soluble polymer and water, forming the combination into a film, and drying the film in a controlled manner, desirably by initially only applying heat to the bottom side of the film, in order to maintain a non-self-aggregating uniform heterogeneity. Desirably, during the initial bottom drying stage, substantially no convection currents, i.e. hot air currents, are permitted to travel across the tops of the films. Once the visco-elastic properties of the film are such that the film components are "locked" in place and cannot move to cause non-uniformity, other methods of heating may then be employed. An active ingredient may be added to the polymer and water combination prior to the drying step. Moreover, the composition desirably is mixed in a manner to minimize the incorporation of air into the mixture and is desirably deaerated, such as by conditioning at room temperature, vacuum treatment or the like, to allow trapped air to escape prior to the drying process. This serves to eliminate bubble and void formation in the final film product, thereby further improving uniformity. Reverse roll is one particularly useful coating technique may also be used to form the film.

In another aspect of the invention, there is a process for preparing a film with a substantially uniform distribution of components. The process includes the steps of combining a water soluble polymer component and water to form a uniformly distributed matrix. This matrix is then formed into a film and fed onto the top side of a substrate surface having top and bottom sides. Heat is applied to the bottom side of the substrate surface in order to dry the film. The matrix from which the film is formed may also include an active ingredient. Also, either alternatively, or in addition to the particular method used to dry the film, the polymer may be selected in order to provide a viscosity that maintains the non-self-aggregating uniform heterogeneity. Reverse roll coating technique may also be used to form the film.

A still further aspect of the present invention provides claim 31

The invention also includes films and a process for preparing films having a substantially uniform distribution of components. The process includes the steps of combining a water soluble polymer component and water to form a uniformly distributed matrix. This matrix is then formed into a film and fed onto a substrate surface having top and bottom sides where the bottom side is in substantially uniform contact with a bottom drying medium, such as a water bath or heated air space controlled at a temperature sufficient to dry the film. Desirably, no external air currents or heat is applied directly to the exposed top surface of the film during the drying process until the film structure has solidified sufficiently to prevent flow, migration and intermolecular attractive forces from creating aggregates or conglomerates. Desirably the heat is controllably conducted by the substrate surface to the film to effectuate drying. The matrix from which the film is formed may also include an active ingredient. Also, either alternatively, or in addition to rapidly drying the film, the polymer may be selected in order to provide a viscosity that maintains the non-self-aggregating uniform heterogeneity.

A pharmaceutical and/or cosmetic dosage form according to the claims is also provided that includes a film having a uniformly dispersed composition including a polymer, a pharmaceutical and/or cosmetic active and a solvent, said film being formed by depositing a wet film of said composition onto a substrate surface and controllably drying the wet film from the side contacting the substrate to prevent self-aggregation and achieve compositional uniformity.

A still further aspect of the present invention includes a pharmaceutical and/or cosmetic dosage form including a polymeric film having no more than a 10% variance of a pharmaceutical and/or cosmetic active per unit area.

The present invention also provides a pharmaceutical composition in the form of a film for external or topical administration, including a composition having a uniformly distributed combination of a polymer, a polar solvent, and a pharmaceutical active, said composition in its dried film form maintaining the uniform distribution of components through the application of controlled bottom drying of the film.

A pharmaceutical dispenser is also provided that includes individual unit dosage forms of the pharmaceutical compositions and films of the present invention. The dosage forms may be optionally stacked in a dispenser or in a roll.

Yet another aspect of the present invention provides an ingestible water-soluble delivery system in the form of a film composition that includes a water-soluble polymer and an anti-foaming or defoaming agent, such as simethicone, which includes a combination of a polymethylsiloxane and silicon dioxide. Simethicone can act as either an anti-foaming or defoaming agent, or both, which reduces or eliminates air from the film composition. An anti-foaming agent will aid in preventing the introduction of air into a composition, while a defoaming agent will aid in removing air from the composition. The composition may also include a pharmaceutical and/or cosmetic active ingredient, flavors, sweeteners, plasticizers, surfactants, or other ingredients to alter the film properties to produce the desired product.

## BRIEF DESCRIPT10N OF THE DRAWINGS

Figure 1 shows a side view of a package containing a unit dosage film of the present invention.

Figure 2 shows a top view of two adjacently coupled packages containing individual unit dosage forms of the present invention, separated by a tearable perforation.

Figure 3 shows a side view of the adjacently coupled packages of Figure 2 arranged in a stacked configuration.

Figure 4 shows a perspective view of a dispenser for dispensing the packaged unit dosage forms, dispenser containing the packaged unit dosage forms in a stacked configuration.

Figure 5 is a schematic view of a roll of coupled unit dose packages of the present invention.

Figure 6 is a schematic view of an apparatus suitable for preparation of a pre-mix, addition of an active, and subsequent formation of the film.

Figure 7 is a schematic view of an apparatus suitable for drying the films of the present invention.

## DETAILED DESCRIPTION OF THE INVENTION

For the purposes of the present invention the term non-self-aggregating uniform heterogeneity refers to the ability of the films of the present invention, which are formed from one or more components in addition to a polar solvent, to provide a substantially reduced occurrence of, i.e. little or no, aggregation or conglomeration of components within the film as is normally experienced when films are formed by conventional drying methods such as a high-temperature air-bath using a drying oven, drying tunnel, vacuum drier, or other such drying equipment. The term heterogeneity, as used in the present invention, includes films that will incorporate a single component, such as a polymer, as well as combinations of components, such as a polymer and an active. Uniform heterogeneity includes the substantial absence of aggregates or conglomerates as is common in conventional mixing and heat drying methods used to form films.

Furthermore, the films of the present invention have a substantially uniform thickness, which is also not provided by the use of conventional drying methods used for drying water-based polymer systems. The absence of a uniform thickness detrimentally affects uniformity of component distribution throughout the area of a given film.

The film products of the present invention are produced by a combination of a properly selected polymer and water, optionally including an active ingredient as well as other fillers known in the art. These films provide a non-self-aggregating uniform heterogeneity of the components within them by utilizing a selected casting or deposition method and a controlled drying process. Examples of controlled drying processes include, but are not limited to, the use of the apparatus disclosed in U.S. Patent No. 4,631,837 to Magoon ("Magoon"), as well as hot air impingement across the bottom substrate and bottom heating plates. Another drying technique for obtaining the films of the present invention is controlled radiation drying, in the absence of uncontrolled air currents, such as infrared and radio frequency radiation (i.e. microwaves).

The objective of the drying process is to provide a method of drying the films that avoids complications, such as the noted "rippling" effect, that are associated with conventional drying methods and which initially dry the upper surface of the film, trapping moisture inside. In conventional oven drying methods, as the moisture trapped inside subsequently evaporates, the top surface is altered by being ripped open and then reformed. These complications are avoided by the present invention, and a uniform film is provided by drying the bottom surface of the film first or otherwise preventing the formation of polymer film formation (skin) on the top surface of the film prior to drying the depth of the film. This may be achieved by applying heat to the bottom surface of the film with substantially no top air flow, or alternatively by the introduction of controlled microwaves to evaporate the water or other polar solvent within the film, again with substantially no top air flow. Yet alternatively, drying may be achieved by using balanced fluid flow, such as balanced air flow, where the bottom and top air flows are controlled to provide a uniform film. In such a case, the air flow directed at the top of the film should not create a condition which would cause movement of particles present in the wet film, due to forces generated by the air currents. Additionally, air currents directed at the bottom of the film should desirably be controlled such that the film does not lift up due to forces from the air. Uncontrolled air currents, either above or below the film, can create non-uniformity in the final film products. The humidity level of the area surrounding the top surface may also be appropriately adjusted to prevent premature closure or skinning of the polymer surface.

This manner of drying the films provides several advantages. Among these are the faster drying times and a more uniform surface of the film, as well as uniform distribution of components for any given area in the film. In addition, the faster drying time allows viscosity to quickly build within the film, further encouraging a uniform distribution of components and decrease in aggregation of components in the final film product. The drying of the film will occur within about five minutes or fewer.

The present invention yields exceptionally uniform film products when attention is paid to reducing the aggregation of the compositional components. By avoiding the introduction of and eliminating excessive air in the mixing process, selecting polymers and solvents to provide a controllable viscosity and by drying the film in a rapid manner from the bottom up, such films result.

The products and processes of the present invention rely on the interaction among various steps of the production of the films in order to provide films that substantially reduce the self-aggregation of the components within the films. Specifically, these steps include the particular method used to form the film, making the composition mixture to prevent air bubble inclusions, controlling the viscosity of the film forming composition and the method of drying the film. More particularly, a greater viscosity of components in the mixture is particularly useful when the active is not soluble in water in order to prevent the active from settling out. However, the viscosity must not be too great as to hinder or prevent the chosen method of casting, which desirably includes reverse roll coating due to its ability to provide a film of substantially consistent thickness.

In addition to the viscosity of the film or film-forming components or matrix, there are other considerations taken into account by the present invention for achieving desirable film uniformity. For example, stable suspensions are achieved which prevent solid (such as drug particles) sedimentation in non-colloidal applications. One approach provided by the present invention is to balance the density of the particulate ($\rho_p$) and the liquid phase ($\rho_1$) and increase the viscosity of the liquid phase ($\mu$). For an isolated particle, Stokes law relates the terminal settling velocity (Vo) of a rigid spherical body of radius (r) in a viscous fluid, as follows:

$$V_o = (2gr^r)(\rho_p - \rho_l)/9\mu$$

At high particle concentrations, however, the local particle concentration will affect the local viscosity and density. The viscosity of the suspension is a strong function of solids volume fraction, and particle-particle and particle-liquid interactions will further hinder settling velocity.

Stokian analyses has shown that the incorporation of a third phase, dispersed air or nitrogen, for example, promotes suspension stability. Further, increasing the number of particles leads to a hindered settling effect based on the solids volume fraction. In dilute particle suspensions, the rate of sedimentation, v, can be expressed as:

$$v/V_o = 1/(1 + \kappa\varphi)$$

where $\kappa$ = a constant, and $\varphi$ is the volume fraction of the dispersed phase. More particles suspended in the liquid phase results in decreased velocity. Particle geometry is also an important factor since the particle dimensions will affect particle-particle flow interactions.

Similarly, the viscosity of the suspension is dependent on the volume fraction of dispersed solids. For dilute suspensions of non-interaction spherical particles, an expression for the suspension viscosity can be expressed as:

$$\mu/\mu_o = 1 + 2.5\phi$$

where $\mu_o$ is the viscosity of the continuous phase and $\phi$ is the solids volume fraction. At higher volume fractions, the viscosity of the dispersion can be expressed as

$$\mu/\mu_o = 1 + 2.5\varphi + C_1\varphi^2 + C_2\varphi^3 + \ldots$$

where C is a constant.

The viscosity of the liquid phase is critical and is desirably modified by customizing the liquid composition to a viscoelastic non-Newtonian fluid with low yield stress values. This is the equivalent of producing a high viscosity continuous phase at rest. Formation of a viscoelastic or a highly structured fluid phase provides additional resistive forces to particle sedimentation. Further, flocculation or aggregation can be controlled minimizing particle-particle interactions. The net effect would be the preservation of a homogeneous dispersed phase.

The addition of hydrocolloids to the aqueous phase of the suspension increases viscosity, may produce viscoelasticity and can impart stability depending on the type of hydrocolloid, its concentration and the particle composition, geometry, size, and volume fraction. The particle size distribution of the dispersed phase needs to be controlled by selecting the smallest realistic particle size in the high viscosity medium, i.e., $<500\mu$m. The presence of a slight yield stress or elastic body at low shear rates may also induce permanent stability regardless of the apparent viscosity. The critical particle diameter can be calculated from the yield stress values. In the case of isolated spherical particles, the maximum shear stress developed in settling through a medium of given viscosity can be given as

$$\tau_{max} = 3V\mu/2r$$

For pseudoplastic fluids, the viscosity in this shear stress regime may well be the zero shear rate viscosity at the Newtonian plateau.

A stable suspension is an important characteristic for the manufacture of a pre-mix composition which is to be fed into the film casting machinery film, as well as the maintenance of this stability in the wet film stage until sufficient drying has occurred to lock-in the particles and matrix into a sufficiently solid form such that uniformity is maintained. For viscoelastic fluid systems, a rheology that yields stable suspensions for extended time period, such as 24 hours, must be balanced with the requirements of highspeed film casting operations. A desirable property for the films is shear thinning or pseudoplasticity, whereby the viscosity decreases with increasing shear rate. Time dependent shear effects such as thixotropy are also advantageous. Structural recovery and shear thinning behavior are important properties, as is the ability for the film to self-level as it is formed.

The rheology requirements for the inventive compositions and films are quite severe. This is due to the need to produce a stable suspension of particles, for example 30-60 wt%, in a viscoelastic fluid matrix with acceptable viscosity values throughout a broad shear rate range. During mixing, pumping, and film casting, shear rates in the range of 10 - $10^5$ sec.$^{-1}$ may be experienced and pseudoplasticity is the preferred embodiment.

In film casting or coating, rheology is also a defining factor with respect to the ability to form films with the desired uniformity. Shear viscosity, extensional viscosity, viscoelasticity, structural recovery will influence the quality of the film. As an illustrative example, the leveling of shear-thinning pseudoplastic fluids has been derived as

$$\alpha^{(n-1/n)} = \alpha_o^{(n-1/n)} - ((n-1)/(2n-1))(\tau/K)^{1/n}\,(2\pi/\lambda)^{(3+n)/n}h^{(2n+1)/n}t$$

where $\alpha$ is the surface wave amplitude, $\alpha_o$ is the initial amplitude, $\lambda$ is the wavelength of the surface roughness, and both "n" and "K" are viscosity power law indices. In this example, leveling behavior is related to viscosity, increasing as n decreases, and decreasing with increasing K.

Desirably, the films or film-forming compositions of the present invention have a very rapid structural recovery, i.e. as the film is formed during processing, it doesn't fall apart or become discontinuous in its structure and compositional uniformity. Such very rapid structural recovery retards particle settling and sedimentation. Moreover, the films or film-forming compositions of the present invention are desirably shear-thinning pseudoplastic fluids. Such fluids with consideration of properties, such as viscosity and elasticity, promote thin film formation and uniformity.

Thus, uniformity in the mixture of components depends upon numerous variables. As described herein, viscosity of the components, the mixing techniques and the rheological properties of the resultant mixed composition and wet casted film are important aspects of the present invention. Additionally, control of particle size and particle shape are further considerations. Desirably, the size of the particulate a particle size of 150 microns or less, for example 100 microns or less. Moreover, such particles may be spherical, substantially spherical, or non-spherical, such as irregularly shaped particles or ellipsoidally shaped particles. Ellipsoidally shaped particles or ellipsoids are desirable because of their ability to maintain uniformity in the film forming matrix as they tend to settle to a lesser degree as compared to spherical particles.

A number of techniques may be employed in the mixing stage to prevent bubble inclusions in the final film. To provide a composition mixture with substantially no air bubble formation in the final product, anti-foaming or surface-tension reducing agents are employed. Additionally, the speed of the mixture is desirably controlled to prevent cavitation of the mixture in a manner which pulls air into the mix. Finally, air bubble reduction can further be achieved by allowing the mix to stand for a sufficient time for bubbles to escape prior to drying the film. Desirably, the inventive process first forms a masterbatch of film-forming components without active ingredients such as drug particles or volatile materials such as flavor oils. The actives are added to smaller mixes of the masterbatch just prior to casting. Thus, the masterbatch pre-mix can be allowed to stand for a longer time without concern for instability in drug or other ingredients.

When the matrix is formed including the film-forming polymer and water in addition to any additives and the active ingredient, this may be done in a number of steps. For example, the ingredients may all be added together or a pre-mix may be prepared. The advantage of a pre-mix is that all ingredients except for the active may be combined in advance, with the active added just prior to formation of the film. This is especially important for actives that may degrade with prolonged exposure to water, air or another polar solvent.

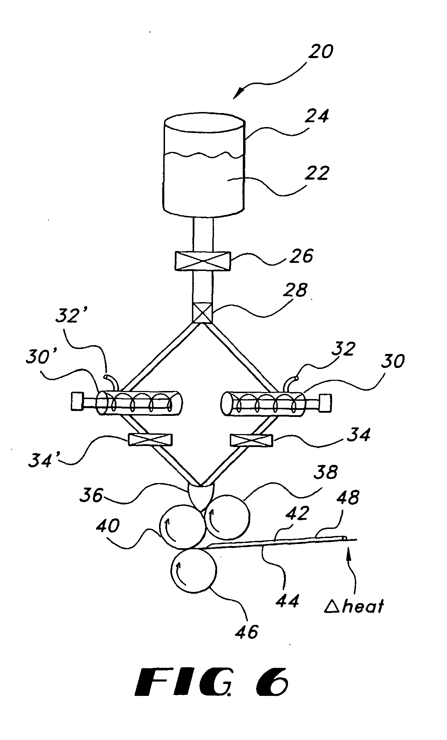

Figure 6 shows an apparatus 20 suitable for the preparation of a pre-mix, addition of an active and subsequent formation of a film. The pre-mix or master batch 22, which includes the film-forming polymer, polar solvent, and any other additives except a drug active is added to the master batch feed tank 24. The components for pre-mix or master batch 22 are desirably formed in a mixer (not shown) prior to their addition into the master batch feed tank 24. Then a pre-determined amount of the master batch is controllably fed via a first metering pump 26 and control valve 28 to either or both of the first and second mixers, 30, 30'. The present invention, however, is not limited to the use of two mixers, 30, 30', and any number of mixers may suitably be used. Moreover, the present invention is not limited to any particular sequencing of the mixers 30, 30', such as parallel sequencing as depicted in Figure 6, and other sequencing or arrangements of mixers, such as series or combination of parallel and series, may suitably be used. The required amount of the drug or other ingredient, such as a flavor, is added to the desired mixer through an opening, 32, 32', in each of the mixers, 30, 30'. Desirably, the residence time of the pre-mix or master batch 22 is minimized in the mixers 30, 30'. While complete dispersion of the drug into the pre-mix or master batch 22 is desirable, excessive residence times may result in leaching or dissolving of the drug, especially in the case for a soluble drug. Thus, the mixers 30, 30' are often smaller, i.e. lower residence times, as compared to the primary mixers (not shown) used in forming the pre-mix or master batch 22. After the drug has been blended with the master batch pre-mix for a sufficient time to provide a uniform matrix, a specific amount of the uniform matrix is then fed to the pan 36 through the second metering pumps, 34, 34'. The metering roller 38 determines the thickness of the film 42 and applies it to the application roller. The film 42 is finally formed on the substrate 44 and carried away via the support roller 46.

While the proper viscosity uniformity in mixture and stable suspension of particles, and casting method are important in the initial steps of forming the composition and film to promote uniformity, the method of drying the wet film is also important. Although these parameters and properties assist uniformity initially, a controlled rapid drying process ensures that the uniformity will be maintained until the film is dry.

The wet film is then dried using controlled bottom drying or controlled microwave drying, desirably in the absence of external air currents or heat on the top (exposed) surface of the film 48 as described herein. Controlled bottom drying or controlled microwave drying advantageously allows for vapor release from the film without the disadvantages of the prior art. Conventional convection air drying from the top is not employed because it initiates drying at the top uppermost portion of the film, thereby forming a barrier against fluid flow, such as the evaporative vapors, and thermal flow, such as the thermal energy for drying. Such dried upper portions serve as a barrier to further vapor release as the portions beneath are dried, which results in non-uniform films. As previously mentioned some top air flow can be used to aid the drying of the films of the present invention, but it must not create a condition that would cause particle movement or a rippling effect in the film, both of which would result in non-uniformity. If top air is employed, it is balanced with the bottom air drying to avoid non-uniformity and prevent film lift-up on the carrier belt. A balance top and bottom air flow may be suitable where the bottom air flow functions as the major source of drying and the top air flow is the minor source of drying. The advantage of some top air flow is to move the exiting vapors away from the film thereby aiding in the overall drying process. The use of any top air flow or top drying, however, must be balanced by a number of factors including, but not limited, to rheological properties of the composition and mechanical aspects of the processing. Any top fluid flow, such as air, also must not overcome the inherent viscosity of the film-forming composition. In other words, the top air flow cannot break, distort or otherwise physically disturb the surface of the composition. Moreover, air velocities are desirably below the yield values of the film, i.e., below any force level that can move the liquids in the film-forming compositions. For thin or low viscosity compositions, low air velocity must be used. For thick or high viscosity compositions, higher air velocities may be used. Furthermore, air velocities are desirable low so as to avoid any lifting or other movement of the film formed from the compositions.

Moreover, the films of the present invention may contain particles that are sensitive to temperature, such as flavors, which may be volatile, or drugs, which may have a low degradation temperature. In such cases, the drying temperature may be decreased while increasing the drying time to adequately dry the uniform films of the present invention. Furthermore, bottom drying also tends to result in a lower internal film temperature as compared to top drying. In bottom drying, the evaporating vapors more readily carry heat away from the film as compared to top drying which lowers the internal film temperature. Such lower internal film temperatures often result in decreased drug degradation and decreased loss of certain volatiles, such as flavors.

Furthermore, particles or particulates may be added to the film-forming composition or matrix after the composition or matrix is cast into a film. For example, particles may be added to the film 42 prior to the drying of the film 42. Particles may be controllably metered to the film and disposed onto the film through a suitable technique, such as through the use of a doctor blade (not shown) which is a device which marginally or softly touches the surface of the film and controllably disposes the particles onto the film surface. Other suitable, but non-limiting, techniques include the use of an additional roller to place the particles on the film surface, spraying the particles onto the film surface, and the like. The particles may be placed on either or both of the opposed film surfaces, i.e., the top and/or bottom film surfaces. Desirably, the particles are securably disposed onto the film, such as being embedded into the film. Moreover, such particles are desirably not fully encased or fully embedded into the film, but remain exposed to the surface of the film, such as in the case where the particles are partially embedded or partially encased.

The particles may be any useful organoleptic agent, cosmetic agent, pharmaceutical agent, or combinations thereof. Desirably, the pharmaceutical agent is a taste-masked or a controlled-release pharmaceutical agent. Useful organoleptic agents include flavors and sweeteners. Useful cosmetic agents include breath freshening or decongestant agents, such as menthol, including menthol crystals.

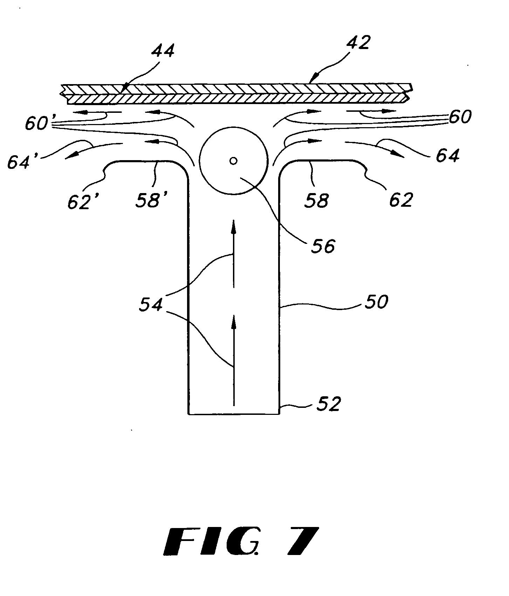

Although the inventive process is not limited to any particular apparatus for the above-described desirable drying, one particular useful drying apparatus 50 is depicted in Figure 7. Drying apparatus 50 is a nozzle arrangement for directing hot fluid, such as but not limited to hot air, towards the bottom of the film 42 which is disposed on substrate 44. Hot air enters the entrance end 52 of the drying apparatus and travels vertically upward, as depicted by vectors 54, towards air deflector 56. The air deflector 56 redirects the air movement to minimize upward force on the film 42. As depicted in Figure 7, the air is tangentially directed, as indicated by vectors 60 and 60', as the air passes by air deflector 56 and enters and travels through chamber portions 58 and 58' of the drying apparatus 50. With the hot air flow being substantially tangential to the film 42, lifting of the film as it is being dried is thereby minimized. While the air deflector 56 is depicted as a roller, other devices and geometries for deflecting air or hot fluid may suitable be used. Furthermore, the exit ends 62 and 62' of the drying apparatus 50 are flared downwardly. Such downward flaring provides a downward force or downward velocity vector, as indicated by vectors 64 and 64', which tend to provide a pulling or drag effect of the film 42 to prevent lifting of the film 42. Lifting of the film 42 may not only result in non-uniformity in the film or otherwise, but may also result in non-controlled processing of the film 42 as the film 42 and/or substrate 44 lift away from the processing equipment.

Monitoring and control of the thickness of the film also contributes to the production of a uniform film by providing a film of uniform thickness. The thickness of the film may be monitored with gauges such as Beta Gauges. A gauge may be coupled to another gauge at the end of the drying apparatus, i.e. drying oven or tunnel, to communicate through feedback loops to control and adjust the opening in the coating apparatus, resulting in control of uniform film thickness.

The film products are generally formed by combining a properly selected polymer and polar solvent, as well as any active ingredient or filler as desired. Desirably, the solvent content of the combination is at least about 30% by weight of the total combination. The matrix formed by this combination is formed into a film, desirably by roll coating, and then dried, desirably by a rapid and controlled drying process to maintain the uniformity of the film, more specifically, a non-self-aggregating uniform heterogeneity. The resulting film will desirably contain less than about 10% by weight solvent, more desirably less than about 8% by weight solvent, even more desirably less than about 6% by weight solvent and most desirably less than about 2%. The solvent is water.

Consideration of the above discussed parameters, such as but not limited to rheology properties, viscosity, mixing method, casting method and drying method, also impact material selection for the different components of the present invention. Furthermore, such consideration with proper material selection provides the compositions of the present invention, including a pharmaceutical and/or cosmetic dosage form or film product having no more than a 10% variance of a pharmaceutical and/or cosmetic active per unit area. In other words, the uniformity of the present invention is determined by the presence of no more than a 10% by weight of pharmaceutical and/or cosmetic variance throughout the matrix. Desirably, the variance is less than 5% by weight, less than 2% by weight, less than 1% by weight, or less than 0.5% by weight.

**Film-Forming Polymers**

The polymer may be water soluble, water swellable, or a combination of one or more either water soluble, water swellable or water insoluble polymers. The polymer may include cellulose or a cellulose derivative. Specific examples of useful water soluble polymers include, but are not limited to, pullulan, hydroxypropylmethyl cellulose, hydroxyethyl cellulose, hydroxypropyl cellulose, polyvinyl pyrrolidone, carboxymethyl cellulose, polyvinyl alcohol, sodium aginate, polyethylene glycol, xanthan gum, tragancanth gum, guar gum, acacia gum, arabic gum, polyacrylic acid, methylmethacrylate copolymer, carboxyvinyl copolymers, starch, gelatin, and combinations thereof.

As used herein the phrase "water soluble polymer" and variants thereof refer to a polymer that is at least partially soluble in water, and desirably fully or predominantly soluble in water, or absorbs water. Polymers that absorb water are often referred to as being water swellable polymers. The materials useful with the present invention may be water soluble or water swellable at room temperature and other temperatures, such as temperatures exceeding room temperature. Moreover, the materials may be water soluble or water swellable at pressures less than atmospheric pressure. Desirably, the water soluble polymers are water soluble or water swellable having at least 20 percent by weight water uptake. Water swellable polymers having a 25 or greater percent by weight water uptake are also useful. Films or dosage forms of the present invention formed from such water soluble polymers are desirably sufficiently water soluble to be dissolvable upon contact with bodily fluids.

Other polymers useful for incorporation into the films of the present invention include biodegradable polymers, copolymers, block polymers and combinations thereof. Among the known useful polymers or polymer classes which meet the above criteria are: poly(glycolic acid) (PGA), poly(lactic acid) (PLA), polydioxanoes, polyoxalates, poly($\alpha$-esters), polyanhydrides, polyacetates, polycaprolactones, poly(orthoesters), polyamino acids, polyaminocarbonates, polyurethanes, polycarbonates, polyamides, poly(alkyl cyanoacrylates), and mixtures and copolymers thereof. Additional useful polymers include, stereopolymers of L- and D-lactic acid, copolymers of bis(p-carboxyphenoxy) propane acid and sebacic acid, sebacic acid copolymers, copolymers of caprolactone, poly(lactic acid)/poly(glycolic acid)/polyethyleneglycol copolymers, copolymers of polyurethane and (poly(lactic acid), copolymers of polyurethane and poly(lactic acid), copolymers of $\alpha$-amino acids, copolymers of $\alpha$-amino acids and caproic acid, copolymers of $\alpha$-benzyl glutamate and polyethylene glycol, copolymers of succinate and poly(glycols), polyphosphazene, polyhydroxy-alkanoates and mixtures thereof. Binary and ternary systems are contemplated.

Other specific polymers useful include those marketed under the Medisorb and Biodel trademarks. The Medisorb materials are marketed by the Dupont Company of Wilmington, Delaware and are generically identified as a "lactide/glycolide co-polymer" containing "propanoic acid, 2-hydroxy-polymer with hydroxy-polymer with hydroxyacetic acid." Four such polymers include lactide/glycolide 100L, believed to be 100% lactide having a melting point within the range of 338°-347°F (170°-175°C); lactide/glycolide 100L, believed to be 100% glycolide having a melting point within the range of 437°-455°F (225°-235°C); lactide/glycolide 85/15, believed to be 85% lactide and 15% glycolide with a melting point within the range of 338°-347°F (170°-175° C); and lactide/glycolide 50/50, believed to be a copolymer of 50% lactide and 50% glycolide with a melting point within the range of 338°-347°F (170°-175°C).

The Biodel materials represent a family of various polyanhydrides which differ chemically.

Although a variety of different polymers may be used, it is desired to select polymers to provide a desired viscosity of the mixture prior to drying. For example, if the active or other components are not soluble in the selected solvent, a polymer that will provide a greater viscosity is desired to assist in maintaining uniformity. On the other hand, if the components are soluble in the solvent, a polymer that provides a lower viscosity may be preferred.

The polymer plays an important role in affecting the viscosity of the film. Viscosity is one property of a liquid that controls the stability of the active in an emulsion, a colloid or a suspension. Generally the viscosity of the matrix will vary from about 400 cps to about 100,000 cps, preferably from about 800 cps to about 60,000 cps, and most preferably from about 1,000 cps to about 40,000 cps. Desirably, the viscosity of the film-forming matrix will rapidly increase upon initiation of the drying process.

The viscosity may be adjusted based on the selected active depending on the other components within the matrix. For example, if the component is not soluble within the selected solvent, a proper viscosity may be selected to prevent the component from settling which would adversely affect the uniformity of the resulting film. The viscosity may be adjusted in different ways. To increase viscosity of the film matrix, the polymer may be chosen of a higher molecular weight or crosslinkers may be added, such as salts of calcium, sodium and potassium. The viscosity may also be adjusted by adjusting the temperature or by adding a viscosity increasing component. Components that will increase the viscosity or stabilize the emulsion/suspension include higher molecular weight polymers and polysaccharides and gums, which include without limitation, alginate, carrageenan, hydroxypropyl methyl cellulose, locust bean gum, guar gum, xanthan gum, dextran, gum arabic, gellan gum and combinations thereof.

It has also been observed that certain polymers which when used alone would ordinarily require a plasticizer to achieve a flexible film, can be combined without a plasticizer and yet achieve flexible films. For example, HPMC and HPC when used in combination provide a flexible, strong film with the appropriate plasticity and elasticity for manufacturing and storage. No additional plasticizer or polyalcohol is needed for flexibility.

## Controlled Release Films

The term "controlled release" is intended to mean the release of active at a preselected or desired rate. This rate will vary depending upon the application. Desirable rates include fast or immediate release profiles as well as delayed, sustained or sequential release. Combinations of release patterns, such as initial spiked release followed by lower levels of sustained release of active are contemplated. Pulsed drug releases are also contemplated.

The polymers that are chosen for the films of the present invention may also be chosen to allow for controlled disintegration of the active. This may be achieved by providing a substantially water insoluble film that incorporates an active that will be released from the film over time. This may be accomplished by incorporating a variety of different soluble or insoluble polymers and may also include biodegradable polymers in combination. Alternatively, coated controlled release active particles may be incorporated into a readily soluble film matrix to achieve the controlled release property of the active inside the digestive system upon consumption.

Films that provide a controlled release of the active are particularly useful for buccal, gingival, sublingual and vaginal applications. The films of the present invention are particularly useful where mucosal membranes or mucosal fluid is present due to their ability to readily wet and adhere to these areas.

The convenience of administering a single dose of a medication which releases active ingredients in a controlled fashion over an extended period of time as opposed to the administration of a number of single doses at regular intervals has long been recognized in the pharmaceutical arts. The advantage to the patient and clinician in having consistent and uniform blood levels of medication over an extended period of time are likewise recognized. The advantages of a variety of sustained release dosage forms are well known. However, the preparation of a film that provides the controlled release of an active has advantages in addition to those well-known for controlled release tablets. For example, thin films are difficult to inadvertently aspirate and provide an increased patient compliance because they need not be swallowed like a tablet. Moreover, certain embodiments of the inventive films are designed to adhere to the buccal cavity and tongue, where they controllably dissolve. Furthermore, thin films may not be crushed in the manner of controlled release tablets which is a problem leading to abuse of drugs such as Oxycontin.

The actives employed in the present invention may be incorporated into the film compositions of the present invention in a controlled release form. For example, particles of drug may be coated with polymers such as ethyl cellulose or polymethacrylate, commercially available under brand names such as Aquacoat ECD and Eudragit E-100, respectively. Solutions of drug may also be absorbed on such polymer materials and incorporated into the inventive film compositions. Other components such as fats and waxes, as well as sweeteners and/or flavors may also be employed in such controlled release compositions.

The actives may be taste-masked prior to incorporation into the film composition, as set forth in co-pending PCT application titled, Uniform Films For Rapid Dissolve Dosage Form Incorporating Taste-Masking Compositions, (based on U.S. Provisional Application No. Express Mail Label No.: EU552991605 US of the same title, filed September 27, 2003, attorney docket No. 1199-15P).

## Actives

When an active is introduced to the film, the amount of active per unit area is determined by the uniform distribution of the film. For example, when the films are cut into individual dosage forms, the amount of the active in the dosage form can be known with a great deal of accuracy. This is achieved because the amount of the active in a given area is substantially identical to the amount of active in an area of the same dimensions in another part of the film. The accuracy in dosage is particularly advantageous when the active is a medicament, i.e. a drug.

The active components that may be incorporated into the films of the present invention include, without limitation pharmaceutical and cosmetic actives, drugs, medicaments, antigens or allergens such as ragweed pollen, spores, microorganisms, seeds, mouthwash components, flavors, fragrances, enzymes, preservatives, sweetening agents, colorants, spices, vitamins and combinations thereof.

A wide variety of medicaments, bioactive active substances and pharmaceutical compositions may be included in the dosage forms of the present invention. Examples of useful drugs include ace-inhibitors, antianginal drugs, anti-arrhythmias, anti-asthmatics, anti-cholesterolemics, analgesics, anesthetics, anti-convulsants, anti-depressants, anti-diabetic agents, anti-diarrhea preparations, antidotes, anti-histamines, anti-hypertensive drugs, anti-inflammatory agents, anti-lipid agents, anti-manics, anti-nauseants, anti-stroke agents, anti-thyroid preparations, anti-tumor drugs, anti-viral agents, acne drugs, alkaloids, amino acid preparations, anti-tussives, anti-uricemic drugs, anti-viral drugs, anabolic preparations, systemic and non-systemic anti-infective agents, anti-neoplastics, anti-parkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anticoagulants, anti-thrombotic drugs, hypnotics, anti-emetics, anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Examples of medicating active ingredients contemplated for use in the present invention include antacids, $H_2$-antagonists, and analgesics. For example, antacid dosages can be prepared using the ingredients calcium carbonate alone or in combination with magnesium hydroxide, and/or aluminum hydroxide. Moreover, antacids can be used in combination with $H_2$-antagonists.

Analgesics include opiates and opiate derivatives, such as oxycodone (available as Oxycontin®), ibuprofen, aspirin, acetaminophen, and combinations thereof that may optionally include caffeine.

Other preferred drugs for other preferred active ingredients for use in the present invention include anti-diarrheals such as immodium AD, anti-histamines, anti-tussives, decongestants, vitamins, and breath fresheners. Common drugs used alone or in combination for colds, pain, fever, cough, congestion, runny nose and allergies, such as acetaminophen, chlorpheniramine maleate, dextromethorphan, pseudoephedrine HCl and diphenhydramine may be included in the film compositions of the present invention.

Also contemplated for use herein are anxiolytics such as alprazolam (available as Xanax®); anti-psychotics such as clozopin (available as Clozaril®) and haloperidol (available as Haldol®); non-steroidal anti-inflammatories (NSAID's) such as dicyclofenacs (available as Voltaren®) and etodolac (available as Lodine®), anti-histamines such as loratadine (available as Claritin®), astemizole (available as Hismanal™), nabumetone (available as Relafen®), and Clemastine (available as Tavist®); anti-emetics such as granisetron hydrochloride (available as Kytril®) and nabilone (available as Cesamet™); bronchodilators such as Bentolin®, albuterol sulfate (available as Proventil®); anti-depressants such as fluoxetine hydrochloride (available as Prozac®), sertraline hydrochloride (available as Zoloft®), and paroxtine hydrochloride (available as Paxil®); anti-migraines such as Imigra®, ACE-inhibitors such as enalaprilat (available as Vasotec®), captopril (available as Capoten®) and lisinopril (available as Zestril®); anti-Alzheimer's agents, such as nicergoline; and $Ca^H$-antagonists such as nifedipine (available as Procardia® and Adalat®), and verapamil hydrochloride (available as Clan®).

Erectile dysfunction therapies include, but are not limited to, drugs for facilitating blood flow to the penis, and for effecting autonomic nervous activities, such as increasing parasympathetic (cholinergic) and decreasing sympathetic (adrenersic) activities. Useful non-limiting drugs include sildenafils, such as Viagra®, tadalafils, such as Cialis®, vardenafils, apomorphines, such as Uprima®, yohimbine hydrochlorides such as Aphrodyne®, and alprostadils such as Caverject®.

The popular $H_2$-antagonists which are contemplated for use in the present invention include cimetidine, ranitidine hydrochloride, famotidine, nizatidien, ebrotidine, mifentidine, roxatidine, pisatidine and aceroxatidine.

Active antacid ingredients include, but are not limited to, the following: aluminum hydroxide, dihydroxyaluminum aminoacetate, aminoacetic acid, aluminum phosphate, dihydroxyaluminum sodium carbonate, bicarbonate, bismuth aluminate, bismuth carbonate, bismuth subcarbonate, bismuth subgallate, bismuth subnitrate, bismuth subsilysilate, calcium carbonate, calcium phosphate, citrate ion (acid or salt), amino acetic acid, hydrate magnesium aluminate sulfate, magaldrate, magnesium aluminosilicate, magnesium carbonate, magnesium glycinate, magnesium hydroxide, magnesium oxide, magnesium trisilicate, milk solids, aluminum mono-ordibasic calcium phosphate, tricalcium phosphate, potassium bicarbonate, sodium tartrate, sodium bicarbonate, magnesium aluminosilicates, tartaric acids and salts.

The pharmaceutically active agents employed in the present invention may include allergens or antigens, such as , but not limited to, plant pollens from grasses, trees, or ragweed; animal danders, which are tiny scales shed from the skin and hair of cats and other furred animals; insects, such as house dust mites, bees, and wasps; and drugs, such as penicillin.

An anti-oxidant may also be added to the film to prevent the degradation of an active, especially where the active is photosensitive.

Cosmetic active agents may include breath freshening compounds like menthol, other flavors or fragrances, especially those used for oral hygiene, as well as actives used in dental and oral cleansing such as quaternary ammonium bases. The effect of flavors may be enhanced using flavor enhancers like tartaric acid, citric acid, vanillin, or the like.

Also color additives can be used in preparing the films. Such color additives include food, drug and cosmetic colors (FD&C), drug and cosmetic colors (D&C), or external drug and cosmetic colors (Ext. D&C). These colors are dyes, their corresponding lakes, and certain natural and derived colorants. Lakes are dyes absorbed on aluminum hydroxide.

Other examples of coloring agents include known azo dyes, organic or inorganic pigments, or coloring agents of natural origin. Inorganic pigments are preferred, such as the oxides or iron or titanium, these oxides, being added in concentrations ranging from about 0.001 to about 10%, and preferably about 0.5 to about 3%, based on the weight of all the components.

Flavors may be chosen from natural and synthetic flavoring liquids. An illustrative list of such agents includes volatile oils, synthetic flavor oils, flavoring aromatics, oils, liquids, oleoresins or extracts derived from plants, leaves, flowers, fruits, stems and combinations thereof. A non-limiting representative list of examples includes mint oils, cocoa, and citrus oils such as lemon, orange, grape, lime and grapefruit and fruit essences including apple, pear, peach, grape, strawberry, raspberry, cherry, plum, pineapple, apricot or other fruit flavors.

The films containing flavorings may be added to provide a hot or cold flavored drink or soup. These flavorings include, without limitation, tea and soup flavorings such as beef and chicken.

Other useful flavorings include aldehydes and esters such as benzaldehyde (cherry, almond), citral i.e., alphacitral (lemon, lime), neral, i.e., beta-citral (lemon, lime), decanal (orange, lemon), aldehyde C-8 (citrus fruits), aldehyde C-9 (citrus fruits), aldehyde C-12 (citrus fruits), tolyl aldehyde (cherry, almond), 2,6-dimethyloctanol (green fruit), and 2-dodecenal (citrus, mandarin), combinations thereof and the like.

The sweeteners may be chosen from the following non-limiting list: glucose (com syrup), dextrose, invert sugar, fructose, and combinations thereof; saccharin and its various salts such as the sodium salt; dipeptide sweeteners such as aspartame; dihydrochalcone compounds, glycyrrhizin; Stevia Rebaudiana (Stevioside); chloro derivatives of sucrose such as sucralose; sugar alcohols such as sorbitol, mannitol, xylitol, and the like. Also contemplated are hydrogenated starch hydrolysates and the synthetic sweetener 3,6-dihydro-6-methyl-1-1-1,2,3-oxathiazin-4-one-2,2-dioxide, particularly the potassium salt (acesulfame-K), and sodium and calcium salts thereof, and natural intensive sweeteners, such as Lo Han Kuo. Other sweeteners may also be used.

When the active is combined with the polymer in the solvent, the type of matrix that is formed depends on the solubilities of the active and the polymer. If the active and/or polymer are soluble in the selected solvent, this may form a solution. However, if the components are not soluble, the matrix may be classified as an emulsion, a colloid, or a suspension.

## Dosages

The film products of the present invention are capable of accommodating a wide range of amounts of the active ingredient. The films are capable of providing an accurate dosage amount (determined by the size of the film and concentration of the active in the original polymer/water combination) regardless of whether the required dosage is high or extremely low. Therefore, depending on the type of active or pharmaceutical composition that is incorporated into the film, the active amount may be as high as about 300mg, desirably up to about 150mg or as low as the microgram range, or any amount therebetween.

The film products and methods of the present invention are well suited for high potency, low dosage drugs. This is accomplished through the high degree of uniformity of the films. Therefore, low dosage drugs, particularly more potent racemic mixtures of actives are desirable.

## Anti-foaming and De-foaming Compositions

Anti-foaming and/or de-foaming components may also be used with the films of the present invention. These components aid in the removal of air, such as entrapped air, from the film-forming compositions. As described above, such entrapped air may lead to non-uniform films. Simethicone is one particularly useful anti-foaming and/or de-foaming agent. The present invention, however, is not so limited and other anti-foam and/or de-foaming agents may suitable be used.

Simethicone is generally used in the medical field as a treatment for gas or colic in babies. Simethicone is a mixture of fully methylated linear siloxane polymers containing repeating units of polydimethylsiloxane which is stabilized with trimethylsiloxy end-blocking unites, and silicon dioxide. It usually contains 90.5-99% polymethylsiloxane and 4-7% silicon dioxide. The mixture is a gray, translucent, viscous fluid which is insoluble in water.

When dispersed in water, simethicone will spread across the surface, forming a thin film of low surface tension. In this way, simethicone reduces the surface tension of bubbles air located in the solution, such as foam bubbles, causing their collapse. The function of simethicone mimics the dual action of oil and alcohol in water. For example, in an oily solution any trapped air bubbles will ascend to the surface and dissipate more quickly and easily, because an oily liquid has a lighter density compared to a water solution. On the other hand, an alcohol/water mixture is known to lower water density as well as lower the water's surface tension. So, any air bubbles trapped inside this mixture solution will also be easily dissipated. Simethicone solution provides both of these advantages. It lowers the surface energy of any air bubbles that trapped inside the aqueous solution, as well as lowering the surface tension of the aqueous solution. As the result of this unique functionality, simethicone has an excellent anti-foaming property that can be used for physiological processes (anti-gas in stomach) as well as any for external processes that require the removal of air bubbles from a product.

In order to prevent the formation of air bubbles in the films of the present invention, the mixing step can be performed under vacuum. However, as soon as the mixing step is completed, and the film solution is returned to the normal atmosphere condition, air will be re-introduced into or contacted with the mixture. In many cases, tiny air bubbles will be again trapped inside this polymeric viscous solution. The incorporation of simethicone into the film-forming composition either substantially reduces or eliminates the formation of air bubbles.

Simethicone may be added to the film-forming mixture as an anti-foaming agent in an amount from about 0.01 weight percent to about 5.0 weight percent, more desirably from about 0.05 weight percent to about 2.5 weight percent, and most desirably from about 0.1 weight percent to about 1.0 weight percent.

**Optional Components**

A variety of other components and fillers may also be added to the films of the present invention. These may include, without limitation, surfactants; plasticizers which assist in compatibilizing the components within the mixture; polyalcohols; anti-foaming agents, such as silicone-containing compounds, which promote a smoother film surface by releasing oxygen from the film; and thermo-setting gels such as pectin, carageenan, and gelatin, which help in maintaining the dispersion of components.

The variety of additives that can be incorporated into the inventive compositions may provide a variety of different functions. Examples of classes of additives include excipients, lubricants, buffering agents, stabilizers, blowing agents, pigments, coloring agents, fillers, bulking agents, sweetening agents, flavoring agents, fragrances, release modifiers, adjuvants, plasticizers, flow accelerators, mold release agents, polyols, granulating agents, diluents, binders, buffers, absorbents, glidants, adhesives, anti-adherents, acidulants, softeners, resins, demulcents, solvents, surfactants, emulsifiers, elastomers and mixtures thereof. These additives may be added with the active ingredient(s).

Useful additives include, for example, gelatin, vegetable proteins such as sunflower protein, soybean proteins, cotton seed proteins, peanut proteins, grape seed proteins, whey proteins, whey protein isolates, blood proteins, egg proteins, acrylated proteins, water-soluble polysaccharides such as alginates, carrageenans, guar gum, agar-agar, xanthan gum, gellan gum, gum arabic and related gums (gum ghatti, gum karaya, gum tragancanth), pectin, water-soluble derivatives of cellulose: alkylcelluloses hydroxyalkylcelluloses and hydroxyalkylalkylcelluloses, such as methylcelulose, hydroxymethylcellulose, hydroxyethylcellulose, hydroxypropylcellulose, hydroxyethylmethylcellulose, hydroxypropylmethylcellulose, hydroxybutylmethylcellulose, cellulose esters and hydroxyalkylcellulose esters such as cellulose acetate phthalate (CAP), hydroxypropylmethylcellulose (HPMC); carboxyalkylcelluloses, carboxyalkylalkylcelluloses, carboxyalkylcellulose esters such as carboxymethylcellulose and their alkali metal salts; water-soluble synthetic polymers such as polyacrylic acids and polyacrylic acid esters, polymethacrylic acids and polymethacrylic acid esters, polyvinylacetates, polyvinylalcohols, polyvinylacetatephthalates (PVAP), polyvinylpyrrolidone (PVP), PVY/vinyl acetate copolymer, and polycrotonic acids; also suitable are phthalated gelatin, gelatin succinate, crosslinked gelatin, shellac, water soluble chemical derivatives of starch, cationically modified acrylates and methacrylates possessing, for example, a tertiary or quaternary amino group, such as the diethylaminoethyl group, which may be quatemized if desired; and other similar polymers.

Such extenders may optionally be added in any desired amount desirably within the range of up to about 80%, desirably about 3% to 50% and more desirably within the range of 3% to 20% based on the weight of all components.

Further additives may be inorganic fillers, such as the oxides of magnesium aluminum, silicon, titanium, etc. desirably in a concentration range of about 0.02% to about 3% by weight and desirably about 0.02% to about 1% based on the weight of all components.

Further examples of additives are plasticizers which include polyalkylene oxides, such as polyethylene glycols, polypropylene glycols, polyethylene-propylene glycols, organic plasticizers with low molecular weights, such as glycerol, glycerol monoacetate, diacetate or triacetate, triacetin, polysorbate, cetyl alcohol, propylene glycol, sorbitol, sodium diethylsulfosuccinate, triethyl citrate, tributyl citrate, and the like, added in concentrations ranging from about 0.5% to about 30%, and desirably ranging from about 0.5% to about 20% based on the weight of the polymer.

There may further be added compounds to improve the flow properties of the starch material such as animal or vegetable fats, desirably in their hydrogenated form, especially those which are solid at room temperature. These fats desirably have a melting point of 50°C or higher. Preferred are tri-glycerides with $C_{12}$-, $C_{14}$-, $C_{16}$-, $C_{18}$-, $C_{20}$- and $C_{22}$- fatty acids. These fats can be added alone without adding extenders or plasticizers and can be advantageously added alone or together with mono- and/or di-glycerides or phosphatides, especially lecithin. The mono- and di-glycerides are desirably derived from the types of fats described above, i.e. with $C_{12}$-, $C_{14}$-, $C_{16}$-, $C_{18}$-, $C_{20}$- and $C_{22}$- fatty acids.

The total amounts used of the fats, mono-, di-glycerides and/or lecithins are up to about 5% and preferably within the range of about 0.5% to about 2% by weight of the total composition

It is further useful to add silicon dioxide, calcium silicate, or titanium dioxide in a concentration of about 0.02% to about 1% by weight of the total composition. These compounds act as texturizing agents.

These additives are to be used in amounts sufficient to achieve their intended purpose. Generally, the combination of certain of these additives will alter the overall release profile of the active ingredient and can be used to modify, i.e. impede or accelerate the release.

Lecithin is one surface active agent for use in the present invention. Lecithin can be included in the feedstock in an amount of from about 0.25% to about 2.00% by weight. Other surface active agents, i.e. surfactants, include, but are not limited to, cetyl alcohol, sodium lauryl sulfate, the Spans™ and Tweens™ which are commercially available from ICI Americas, Inc. Ethoxylated oils, including ethoxylated castor oils, such as Cremophor® EL which is commercially available from BASF, are also useful. Carbowax™ is yet another modifier which is very useful in the present invention. Tweens™ or combinations of surface active agents may be used to achieve the desired hydrophilic-lipophilic balance ("HLB"). The present invention, however, does not require the use of a surfactant and films or film-forming compositions of the present invention may be essentially free of a surfactant while still providing the desirable uniformity features of the present invention.

As additional modifiers which enhance the procedure and product of the present invention are identified, Applicants intend to include all such additional modifiers within the scope of the invention claimed herein.

Other ingredients include binders which contribute to the ease of formation and general quality of the films. Non-limiting examples of binders include starches, pregelatinize starches, gelatin, polyvinylpyrrolidone, methylcellulose, sodium carboxymethylcellulose, ethylcellulose, polyacrylamides, polyvinyloxoazolidone, and polyvinylalcohols.

## Forming the Film

The films of the present invention must be formed into a sheet prior to drying. After the desired components are combined to form a multi-component matrix, including the polymer, water, and an active or other components as desired, the combination is formed into a sheet or film, by any method known in the art such as extrusion, coating, spreading, casting or drawing the multi-component matrix. If a multi-layered film is desired, this may be accomplished by co-extruding more than one combination of components which may be of the same or different composition. A multi-layered film may also be achieved by coating, spreading, or casting a combination onto an already formed film layer.

Although a variety of different film-forming techniques may be used, it is desirable to select a method that will provide a flexible film, such as reverse roll coating. The flexibility of the film allows for the sheets of film to be rolled and transported for storage or prior to being cut into individual dosage forms. Desirably, the films will also be self-supporting or in other words able to maintain their integrity and structure in the absence of a separate support. Furthermore, the films of the present invention may be selected of materials that are edible or ingestible.

Coating or casting methods are particularly useful for the purpose of forming the films of the present invention. Specific examples include reverse roll coating, gravure coating, immersion or dip coating, metering rod or meyer bar coating, slot die or extrusion coating, gap or knife over roll coating, air knife coating, curtain coating, or combinations thereof, especially when a multi-layered film is desired.

Roll coating, or more specifically reverse roll coating, is particularly desired when forming films in accordance with the present invention. This procedure provides excellent control and uniformity of the resulting films, which is desired in the present invention. In this procedure, the coating material is measured onto the applicator roller by the precision setting of the gap between the upper metering roller and the application roller below it. The coating is transferred from the application roller to the substrate as it passes around the support roller adjacent to the application roller. Both three roll and four roll processes are common.

The gravure coating process relies on an engraved roller running in a coating bath, which fills the engraved dots or lines of the roller with the coating material. The excess coating on the roller is wiped off by a doctor blade and the coating is then deposited onto the substrate as it passes between the engraved roller and a pressure roller.

Offset Gravure is common, where the coating is deposited on an intermediate roller before transfer to the substrate.

In the simple process of immersion or dip coating, the substrate is dipped into a bath of the coating, which is normally of a low viscosity to enable the coating to run back into the bath as the substrate emerges.

In the metering rod coating process, an excess of the coating is deposited onto the substrate as it passes over the bath roller. The wire-wound metering rod, sometimes known as a Meyer Bar, allows the desired quantity of the coating to remain on the substrate. The quantity is determined by the diameter of the wire used on the rod.

In the slot die process, the coating is squeezed out by gravity or under pressure through a slot and onto the substrate. If the coating is 100% solids, the process is termed "Extrusion" and in this case, the line speed is frequently much faster than the speed of the extrusion. This enables coatings to be considerably thinner than the width of the slot.

The gap or knife over roll process relies on a coating being applied to the substrate which then passes through a "gap" between a "knife" and a support roller. As the coating and substrate pass through, the excess is scraped off.

Air knife coating is where the coating is applied to the substrate and the excess is "blown off" by a powerful jet from the air knife. This procedure is useful for aqueous coatings.

In the curtain coating process, a bath with a slot in the base allows a continuous curtain of the coating to fall into the gap between two conveyors. The object to be coated is passed along the conveyor at a controlled speed and so receives the coating on its upper face.

### Drying the Film

The drying step is also a contributing factor with regard to maintaining the uniformity of the film composition. A controlled drying process is particularly important when, in the absence of a viscosity increasing composition or a composition in which the viscosity is controlled, for example by the selection of the polymer, the components within the film may have an increased tendency to aggregate or conglomerate. An alternative method of forming a film with an accurate dosage, that would not necessitate the controlled drying process, would be to cast the films on a predetermined well. With this method, although the components may aggregate, this will not result in the migration of the active to an adjacent dosage form, since each well may define the dosage unit per se.

When a controlled or rapid drying process is desired, this may be through a variety of methods. A variety of methods may be used including those that require the application of heat. The liquid carriers are removed from the film in a manner such that the uniformity, or more specifically, the non-self-aggregating uniform heterogeneity, that is obtained in the wet film is maintained.

The film is dried from the bottom of the film to the top of the film. Desirably, substantially no air flow is present across the top of the film during its initial setting period, during which a solid, visco-elastic structure is formed. This takes place within the first few minutes, i.e. the first 0.5 to 4.0 minutes of the drying process. Controlling the drying in this manner, prevents the destruction and reformation of the film's top surface, which results from conventional drying methods. This is accomplished by forming the film and placing it on the top side of a surface having top and bottom sides. Then, heat is initially applied to the bottom side of the film to provide the necessary energy to evaporate or otherwise remove the liquid carrier. The films dried in this manner dry more quickly and evenly as compared to air-dried films, or those dried by conventional drying means. In contrast to an air-dried film that dries first at the top and edges, the films dried by applying heat to the bottom dry simultaneously at the center as well as at the edges. This also prevents settling of ingredients that occurs with films dried by conventional means.

The temperature at which the films are dried is 100°C or less, desirably about 90°C or less, and most desirably about 80°C or less.

Another method of controlling the drying process, which may be used alone or in combination with other controlled methods as disclosed above includes controlling and modifying the humidity within the drying apparatus where the film is being dried. In this manner, the premature drying of the top surface of the film is avoided.

Additionally, it has also been discovered that the length of drying time can be properly controlled, i.e. balanced with the heat sensitivity and volatility of the components, and particularly the flavor oils and drugs. The amount of energy, temperature and length and speed of the conveyor can be balanced to accommodate such actives and to minimize loss, degradation or ineffectiveness in the final film.

A specific example of an appropriate drying method is that disclosed by Magoon. Magoon is specifically directed toward a method of drying fruit pulp. However, the present inventors have adapted this process toward the preparation of thin films.

The method and apparatus of Magoon are based on an interesting property of water. Although water transmits energy by conduction and convection both within and to its surroundings, water only radiates energy within and to water. Therefore, the apparatus of Magoon includes a surface onto which the fruit pulp is placed that is transparent to infrared radiation. The underside of the surface is in contact with a temperature controlled water bath. The water bath temperature is desirably controlled at a temperature slightly below the boiling temperature of water. When the wet fruit pulp is placed on the surface of the apparatus, this creates a "refractance window." This means that infrared energy is permitted to radiate through the surface only to the area on the surface occupied by the fruit pulp, and only until the fruit pulp is dry. The apparatus of Magoon provides the films of the present invention with an efficient drying time reducing the instance of aggregation of the components of the film.

The films may initially have a thickness of about 500 $\mu$m to about 1,500 $\mu$m, or about 20 mils to about 60 mils, and when dried have a thickness from about 3 $\mu$m to about 250 $\mu$m, or about 0.1mils to about 10mils. Desirably, the dried films will have a thickness of about 2 mils to about 8 mils, and more desirably, from about 3 mils to about 6 mils.

### Uses of Thin Films

The thin films of the present invention are well suited for many uses. The high degree of uniformity of the components of the film makes them particularly well suited for incorporating pharmaceuticals. Furthermore, the polymers used in construction of the films may be chosen to allow for a range of disintegration times for the films. A variation or extension in the time over which a film will disintegrate may achieve control over the rate that the active is released, which may allow for a sustained release delivery system. In addition, the films may be used for the administration of an active to any of several body surfaces, especially those including mucous membranes, such as oral, anal, vaginal, ophthalmological, the surface of a wound, either on a skin surface or within a body such as during surgery, and similar surfaces.

The films may be used to orally administer an active. This is accomplished by preparing the films as described above and introducing them to the oral cavity of a mammal. This film may be prepared and adhered to a second or support layer from which it is removed prior to use, i.e. introduction to the oral cavity. An adhesive may be used to attach the film to the support or backing material which may be any of those known in the art, and is preferably not water soluble. If an adhesive is used, it will desirably be a food grade adhesive that is ingestible and does not alter the properties of the active. Mucoadhesive compositions are particularly useful. The film compositions in many cases serve as mucoadhesives themselves.

The films may be applied under or to the tongue of the mammal. When this is desired, a specific film shape, corresponding to the shape of the tongue may be preferred. Therefore the film may be cut to a shape where the side of the film corresponding to the back of the tongue will be longer than the side corresponding to the front of the tongue. Specifically, the desired shape may be that of a triangle or trapezoid. Desirably, the film will adhere to the oral cavity preventing it from being ejected from the oral cavity and permitting more of the active to be introduced to the oral cavity as the film dissolves.

Another use for the films of the present invention takes advantage of the films' tendency to dissolve quickly when introduce to a liquid. An active may be introduced to a liquid by preparing a film in accordance with the present invention, introducing it to a liquid, and allowing it to dissolve. This may be used either to prepare a liquid dosage form of an active, or to flavor a beverage.

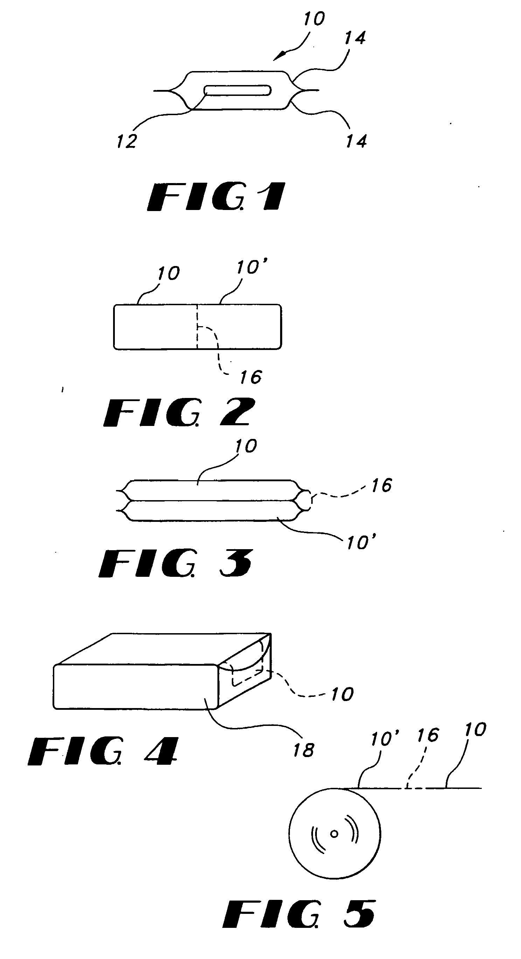

The films of the present invention are desirably packaged in sealed, air and moisture resistant packages to protect the active from exposure oxidation, hydrolysis, volatilization and interaction with the environment. Referring to Figure 1, a packaged pharmaceutical dosage unit 10, includes each film 12 individually wrapped in a pouch or between foil and/or plastic laminate sheets 14. As depicted in Figure 2, the pouches 10, 10' can be linked together with tearable or perforated joints 16. The pouches 10, 10'may be packaged in a roll as depicted in Figure 5 or stacked as shown in Figure 3 and sold in a dispenser 18 as shown in Figure 4. The dispenser may contain a full supply of the medication typically prescribed for the intended therapy, but due to the thinness of the film and package, is smaller and more convenient than traditional bottles used for tablets, capsules and liquids. Moreover, the films of the present invention dissolve instantly upon contact with saliva or mucosal membrane areas, eliminating the need to wash the dose down with water.

Desirably, a series of such unit doses are packaged together in accordance with the prescribed regimen or treatment, e.g., a 10-90 day supply, depending on the particular therapy. The individual films can be packaged on a backing and peeled off for use.

The features and advantages of the present invention are more fully shown by the following examples which are provided for purposes of illustration.

## EXAMPLES

### Examples A-I:

Water soluble thin film compositions of the present invention are prepared using the amounts described in Table 1.

**TABLE 1**

| Ingredient | Weight (g) | | | | | | | | |
|---|---|---|---|---|---|---|---|---|---|
| | A | B | C | D | E | F | G | H | I |
| Hydroxypropylmethyl cellulose | | 1.76 | | 1.63 | 32.00 | | 3.67 | | 32.00 |
| Peppermint oil | | 0.90 | 1.0 | 1.05 | | 8.0 | 2.67 | | |
| Sweetener | 0.15 | 0.15 | 0.22 | 0.10 | | 4.6 | 1.53 | 0.15 | |
| Polyvinylpyrrolidone | | 0.94 | | 1.05 | | 7.0 | 2.33 | | |
| Tween 80[1] | 0.5 | 0.5 | 2.0 | 0.65 | 11.80 | | 1.35 | 0.5 | 11.80 |

(continued)

| Ingredient | Weight (g) | | | | | | | | |
|---|---|---|---|---|---|---|---|---|---|
| | A | B | C | D | E | F | G | H | I |
| Simethicone[2] | 0.2 | 0.2 | 0.15 | 0.30 | 1.80 | | 0.21 | 0.2 | 1.80 |
| Listerine[3] | 83.35 | | | | | | | 83.35 | |
| Methylcellulose | 6.0 | | | | | | | | |
| Cornstarch[4] | | | 1.75 | | | | | | |
| Agar | | | 1.25 | | | | | | |
| Water | | 42.24 | 93.63 | 39.22 | 768.0 | 280.0 | 88.24 | | 768.0 |
| Loratadine[5] | | | | | 19.2 | | | | 19.2 |
| Pullulan[6] | | | | | | | | 6.0 | |
| Ibuprofen | | | | | | | | | 38.4 |
| [1]Available from ICI Americas [2]Available from OSI [3]Available from Pfizer, Inc. including thymol (0.064%), eucalyptol (0.092%), methyl salicylate (0.060%), menthol (0.042%), water (up to 72.8%), alcohol (26.9%), benzoic acid, poloxamer 407, sodium benzoate, and caramel color [4]Available from Grain Processing Corporation as Pure Cote B792 [5]Available from Schering Corporation as Claritin [6]Available from Hayashibara Biochemical Laboratories, Inc., Japan | | | | | | | | | |

The ingredients of inventive compositions A-I were combined by mixing until a uniform mixture was achieved. The compositions were then formed into a film by reverse roll coating. These films were then dried on the top side of an infrared transparent surface, the bottom side of which was in contact with a heated water bath at approximately 99°C. No external thermal air currents were present above the film. The films were dried to less than about 6% by weight water in about 4 to 6 minutes. The films were flexible, self-supporting and provided a uniform distribution of the components within the film.

The uniform distribution of the components within the film was apparent by examination by either the naked eye or under slight magnification. By viewing the films it was apparent that they were substantially free of aggregation, i.e. the carrier and the actives remained substantially in place and did not move substantially from one portion of the film to another. Therefore, there was substantially no disparity among the amount of active found in any portion of the film.

Uniformity was also measured by first cutting the film into individual dosage forms. Twenty-five dosage forms of substantially identical size were cut from the film of inventive composition (E) above from random locations throughout the film. Then eight of these dosage forms were randomly selected and additively weighed. The additive weights of eight randomly selected dosage forms, are as shown in Table 2 below:

### TABLE 2

| Sample | Additive Weight (g) | |
|---|---|---|
| | Trial 1 | Trial 2 |
| 1 | 0.04 | 0.04 |
| 2 | 0.08 | 0.08 |
| 3 | 0.12 | 0.12 |
| 4 | 0.16 | 0.16 |
| 5 | 0.20 | 0.20 |
| 6 | 0.24 | 0.24 |
| 7 | 0.28 | 0.28 |
| 8 | 0.32 | 0.32 |

The individual dosages were consistently 0.04gm, which shows that the distribution of the components within the film was consistent and uniform. This is based on the simple principal that each component has a unique density. Therefore, when the components of different densities are combined in a uniform manner in a film, as in the present invention, individual dosages forms from the same film of substantially equal dimensions, will contain the same mass.

An alternative method of determining the uniformity of the active is to cut the film into individual doses. The individual doses may then be dissolved and tested for the amount of active in films of particular size. This demonstrates that films of substantially similar size cut from different locations on the same film contain substantially the same amount of active.

When the films formed from inventive compositions A-H are placed on the tongue, they rapidly dissolve, releasing the active ingredient. Similarly, when they are placed in water, the films rapidly dissolve which provides a flavored drink when the active is chosen to be a flavoring.

### Examples J-L:

Thin films that have a controlled degradation time and include combinations of water soluble and water insoluble polymers and water soluble films that allow controlled release of an active are prepared using approximately the amounts described in Table 3.

**TABLE 3**

|  | Weight (g) | | |
|---|---|---|---|
| Ingredient | J | K | L |
| Hydroxypropylmethyl cellulose |  | 1.0 | 1.0 |
| Tween 80[1] | 0.7 | 0.7 | 0.7 |
| Water |  |  | 5.0 |
| Aquacoat ECD[2] | 17.0 | 17.0 | 17.5 |
| Peppermint oil | 1.0 | 0.4 | 1.1 |
| [1]Available from ICI Americas<br>[2] A 30% by weight aqueous dispersion of ethyl cellulose available from FMC | | | |

The components of inventive compositions J-L were combined and formed into films using the methods for preparing inventive compositions A-I above. These films were also flexible, self-supporting and provided a uniform distribution of active which permits accuracy in dosing.

The uniformity of the films prepared from inventive compositions J-L may also be tested by either visual means measuring the weights of individual dosage films, or by dissolving the films and testing for the amount of active as described above.

### Examples M-O:

An alternative method of preparing films which provides an accurate dosing may be used for any of inventive compositions A-I. The method begins with first combining the ingredients with mixing. The combination of ingredients is then divided among individual wells or molds. In such a method, aggregation of the components during drying is prevented by the individual wells.

**TABLE 4**

|  | Weight % | | |
|---|---|---|---|
| Ingredient | M | N | O |
| 5% Methylcellulose Solution[1] | 73.22 | 44.22 | 74.22 |
| Raspberry Flavor | 3.28 | 3.28 | 3.28 |
| Sweetener Blends | 1.07 | 1.07 | 1.07 |
| Tween-80[2] | 2.47 | 2.47 | 2.47 |

(continued)

| Ingredient | Weight % | | |
| --- | --- | --- | --- |
| | M | N | O |
| Polyvinylpyrrolidone | 3.30 | 3.30 | 3.30 |
| Ethanol 95% | 8.24 | 8.24 | 8.24 |
| Propylene Glycol | 1.65 | 1.65 | 1.65 |
| Calcium Carbonate | 4.12 | 4.12 | 4.12 |
| Cornstarch[3] | 1.65 | 1.65 | 1.65 |
| Red Dye[4] | 1.00 | | |
| Corn Syrup[5] | | 30.00 | |
| [1] Available from Dow Chemical Co. as Methocel K35 [2] Available from ICI Americas [3] Available from Grain Processing Corporation as Pure Cote B792 [4] Available from McCormick [5] Available from Bestfoods, Inc. as Karo Syrup | | | |

The ingredients in the above Table 4 were combined and formed into a film by casting the combination of ingredients onto the glass surface and applying heat to the bottom side of the glass. This provided inventive compositions M-O.

The film of composition M was examined both prior to and after drying for variations in the shading provided by the red dye. The film was examined both under sunlight and by incandescent bulb light. No variations in shade or intensity of color were observed.

Further testing of the films of composition M included testing of absorption which is directly related to concentration. The film was cut into segments each measuring 1.0 in by 0.75 in., which were consecutively assigned numbers. Approximately 40 mg of the scrap material from which the segments were cut was dissolved in about 10 ml of distilled water and then quantitatively transferred to a 25 ml volumetric flask and brought to volume. The solution was centrifuged and scanned at 3nm intervals from 203-1200nm. The frequency of maximum absorption was found to be 530nm. The solution was then re-centrifuged at a higher RPM (for the same length of time) and re-scanned, which demonstrated no change in the % transmission or frequency.

Each of the segments were weighed to 0.1 mg and then dissolved in 10ml distilled water and transferred quantitatively to a 25 ml volumetric flask and brought to volume with distilled water. Each segment solution was then centrifuged as above, and then scanned, at first from 203-1200nm and later from only 500nm to 550nm at a 1nm scanning speed. The value recorded was the % transmission at the lowest wave length, which was most frequently 530nm.

The absorption values are shown in Table 5 below:

**TABLE 5**

| Segment | mg / % A |
| --- | --- |
| 1-2 | 1.717 |
| 3-4 | 1.700 |
| 5-6 | 1.774 |
| 7* | 1.701 |
| 9-10 | 1.721 |
| 11-12 | 1.729 |
| 13-14 | 1.725 |
| 15-16 | 1.713 |
| * segment 8 was lost | |

The overall average absorption was 1.724. Of the 15 segments tested, the difference between the highest and lowest values was 0.073 units, or 4% based on the average. This shows excellent control over the uniformity of the dye within the composition because the absorption is directly proportional to the concentration of the dye within each segment.

The film of inventive composition N provided a very flexible film. This film was able to be stretched and exhibited a very high tensile strength.

After forming the film of inventive composition O, the film was removed from the glass by very rapidly stripping the length of the glass with a razor. This provided very tightly wound "toothpick-like" dosage forms. Each dosage form consistently weighed 0.02 g. This demonstrates the uniformity of the dosage forms as well as the superior self-supporting properties of the films.

Examples P-W:

Compositions P-W were prepared to demonstrate the interaction among various conditions in production of films as they relate to the present invention. The ingredients in the below Table 6 were combined and formed into a film using the process parameters listed in Table 7 below, prepared in a 6m drying tunnel designed to incorporate bottom drying of the films. Each of the examples shows the effect of different ingredient formulations and processing techniques on the resultant film products.

**TABLE 6**

| | Weight (g) | | | | | | | |
|---|---|---|---|---|---|---|---|---|
| Ingredient | P | Q | R | S | T | U | V | W |
| Hydroxypropylmethyl cellulose | 320 | 320 | 320 | 320 | 320 | 320 | 345 | 345 |
| Water | 1440 | 1440 | 1440 | 1440 | | 1440 | 999 | 999 |
| Sweetener | | | | | | 60 | 60 | 45 |
| Mint Flavor | | | | | | 80 | 80 | |
| Propylene Glycol | 50 | 50 | 50 | 100 | 100 | 100 | 100 | 69.3 |
| Xanthan | 22 | | 11 | 11.23 | 10 | 10 | 10 | 6.9 |
| Water/Ethanol(60/40) | | | | | 1440 | | | |
| Orange Flavor | | | | | | | | 42 |

**TABLE 7**

| | | Film Thickness (Micron) | Top[1] v (m/sec) | Bot.[1] v (m/sec) | T[1] (°C) | Top[2] v (m/sec) |
|---|---|---|---|---|---|---|
| | | | | | | |
| | P1 | 100 | 0 | 22 | 75 | 0 |
| | P2 | 350 | 0 | 22 | 75 | 0 |
| | P3 | 350 | 0 | 40 | 75 | 0 |
| | P4 | 350 | 0 | 40 | 75 | 0 |
| | P5 | 350 | 10 | 40 | 75 | 10 |
| | | | | | | |
| | Q | 350 | 0 | 40 | 75 | 10 |
| | | | | | | |
| | R | 350 | 0 | 40 | 85 | 10 |
| | | | | | | |
| | S1 | 250 | 0 | 40 | 100 | 0 |

(continued)

| | Film Thickness (Micron) | Top[1] v (m/sec) | Bot.[1] v (m/sec) | T[1] (°C) | Top[2] v (m/sec) |
|---|---|---|---|---|---|
| S2 | 300 | 0 | 40 | 100 | 0 |
| S3 | 350 | 0 | 40 | 100 | 0 |
| | | | | | |
| T1 | 250 | 0 | 40 | 100 | 0 |
| T2 | 350 | 0 | 40 | 100 | 0 |
| | | | | | |
| U1 | 300 | 0 | 40 | 100 | 0 |
| U2 | 250 | 0 | 40 | 100 | 0 |
| U3 | 300 | 0 | 40 | 100 | 0 |
| V1 | 300 | 0 | 40 | 100 | 0 |
| V2 | 300 | 0 | 40 | 100 | 0 |
| V3 | 300 | 0 | 40 | 100 | 0 |
| | | | | | |
| W1 | 300 | 0 | 40 | 93 | 0 |
| W2 | 250 | 0 | 40 | 90 | 0 |
| W3 | 200 | 0 | 40 | 90 | 0 |
| | Bot.[2] v (m/sec) | T[2] (°C) | Film Weight (g) | Coater Speed m/min | % Moisture |
| | | | | | |
| P1 | 23 | 60 | 109 | 5 | >20 |
| P2 | 23 | 60 | n/a | 5 | >20 |
| P3 | 40 | 60 | 161 | 3 | >20 |
| P4 | 40 | 75 | 191 | 3 | >20 |
| P5 | 40 | 75 | 253 | 3 | >20 |
| | | | | | |
| Q | 40 | 75 | n/a | 3 | >20 |
| | | | | | |
| R | 0 | 85 | | 2.5 | >20 |
| | | | | | |
| S1 | 40 | 90 | 163 | 1.5 | <5 |
| S2 | 40 | 90 | 193 | 1.5 | <5 |
| S3 | 40 | 90 | 225 | 1.5 | <5 |
| | | | | | |
| T1 | 40 | 90 | 64 | 1.5 | <5 |
| T2 | 40 | 90 | 83 | 1.5 | <5 |
| | | | | | |
| U1 | 40 | 90 | 208 | 1.5 | 20 |

(continued)

|  | Bot.[2] v (m/sec) | T[2] (°C) | Film Weight (g) | Coater Speed m/min | % Moisture |
|---|---|---|---|---|---|
| U2 | 40 | 90 | 177 | 1.5 | 20 |
| U3 | 40 | 90 | 212 | 1.3 | 20 |
| V1 | 40 | 90 | 237 | 1.3 | 20 |
| V2 | 40 | 100 | 242 | 1.3 | 20 |
| V3 | 40 | 100 | 221 | 1 | 6 |
|  |  |  |  |  |  |
| W1 | 40 | 90 | 220 | 1.3 | 5 |
| W2 | 40 | 90 | 199 | 1.3 | 5 |
| W3 | 40 | 90 | 169 | 1.3 | 5 |
| [1] First Heater Section (3m) <br> [2] Second Heater Section (3m) | | | | | |

In Table 7, each of the process parameters contributes to different properties of the films. Film thickness refers to the distance between the blade and the roller in the reverse roll coating apparatus. Bottom velocity and top velocity refer to the speed of air current on the bottom and top sides of the film, respectively. The film weight is a measure of the weight of a circular section of the substrate and the film of 100 cm$^2$.

Compositions P-R show the effects of visco-elastic properties on the ability to coat the film composition mixture onto the substrate for film formation. Composition P displayed a stringy elastic property. The wet film would not stay level, the coating was uneven, and the film did not dry. In Composition Q, substantially the same formulation as P was used however the xanthan was not included. This product coated the substrate but would not stay level due to the change in the visco-elastic properties of the wet foam. Composition R was prepared using substantially the same formulation, but incorporated one-half of the amount of xanthan of Composition P. This formulation provided a composition that could be evenly coated. Compositions P-Q demonstrate the importance of proper formulation on the ability of the film matrix to conform to a particular coating technique.

The films produced from Composition S contained a large amount of air in the films. This is shown by the dried film thickness which was the same despite that variation in the coated thickness as in Table 7. Microscopic examination of the film revealed a large number of air bubbles in the film. In order to correct for the addition of air in the films, care must be taken in the mixing process to avoid air inclusion.

Composition T included a change in the solvent to 60/40 water ethanol. Composition T was stirred slowly for 45min. to deaerate the mixture. The dried weight film products T1 and T2 were consistent with the increase in solids from T1 to T2. The films dried much faster with less than 5% moisture. With the particular combination of ingredients in Composition T, the substitution of part ethanol for part water allowed the film to dry more quickly. The elimination of air from the film as a result of the slow stirring also contributed to the uniformity of the final film product and the faster drying time.

Only water was used as a solvent in Composition U. The dried weight of the U1-U3 changed consistently in accordance with the change in coating thickness indicating that no air bubbles were present. However, these films contained 20% moisture upon exit from the oven, unlike the films of Composition T, which included part ethanol and dried completely.

The amount of solids was increased and the amount of water was decreased in Compositions V1 and V2. The dried weight was greater than U1-U3 due to the increase in solids, however the films still contained 20% moisture upon exit from the oven, similar to Composition U.

The coating line speed was reduced for Composition V3, to prevent premature drying of the exposed top film surface. This film product dried to 6% moisture.

While increasing the amount of solids improved the film weight, longer drying times were required. This was due to the surface of the film sealing preventing easy removal of the water. Therefore, for Compositions W1-W3, the temperature in the first 3m section of the dryer was decreased. This prevented the premature drying of the top surface of the films. Even at greater film thicknesses, the films were dried to 5% moisture even at faster coater line speeds.

**Examples X-AA:**

**TABLE 8**

| Ingredient | Weight (g) | | | |
|---|---|---|---|---|
| | X | Y | Z | AA |
| Loratadine | 104.69 | | | |
| Zomig | | 52.35 | | |
| Paxil | | | 104.69 | |
| Hydroxypropyl methylcellulose | 320 | 320 | 320 | 150 |
| Sweetener blend | 60 | 60 | 60 | 0.4 |
| Simethicone | 1.5 | 1.5 | 1.5 | 1.5 |
| Propylene glycol | 100 | 100 | 100 | |
| Water | 1440 | 1440 | 1440 | 790 |
| Cream essence | | | | 0.4 |
| Polyvinyl pyrrolidinone | | | | 4 |
| Ethanol | | | | 40 |
| Cocoa | | | | 55.2 |
| Polyoxyl-40-stearate | | | | 7 |

Compositions X, Y and Z of Table 8 were taste mask coated using a Glatt coater and Eudragit E-100 polymethacrylate polymer as the coating. The coating was spray coated at a 20% level. Therefore 10mg of drug 12.5 mg of the final dry product must be weighed.

The base formula which excluded the drug additive was mixed with care to not incorporate air. After initial mixing the formula was slowly mixed to deaerate over 30 min. During this time the drug was weighed and prepared for addition to the base mix.

For Composition X, the Loratadine (80% drug) was added slowly to the mix with stirring. After 5 min. of stirring, the total mix was added to the pan of a three roll coater set (reverse roll coater) at 30 micron coating thickness.

The process bottom temperature was set at 90°C with no top heat or air, the bottom air velocity was set at 40 m/sec., and the line speed was set at 1.3 m/min. Total drying time for the film was 4.6 min.

The liquid was coated at 30 microns and dried in the oven in less than 5 min. The film was flexible and a 1" x .75" piece weighed 70 mg and contained 10 mg of Loratadine.

The experiment was repeated for Compositions Y and Z, Zomig and Paxil, respectively. Both produced flexible films with the target weight of 70 mg containing 5 mg of Zomig and 70 mg containing 10 mg of Paxil, respectively.

The products were sweet without any noticeable drug aftertaste.

The ingredients of Composition AA were mixed in order to reduce air captured in the fluid matrix. After mixing 45 g of loratadine coated at a 80% active level and 20% coating using Eudragit E-100, this mixture was added slowing with mixing until the drug was evenly dispersed, approximately 5 min. The liquid was then deposited into the 3 roll coater (reverse roll coater) and coated at 30 microns at a line speed of 1.3 m/min. The oven temperature was set at 90°C to apply air and heat to the bottom only, with an air velocity set at 40 m/sec. The dried film was 0.005 inch. thick (5 mil) and was cut into 1 in. x 0.75 in. pieces weighing 70 mg +/- 0.7 mg, demonstrating the uniformity of the composition of the film. The film was flexible with 5% moisture, free of air bubbles, and had uniform drug distribution as seen under the light microscope, as well as shown by the substantially identical weight measurements of the film pieces.

**Examples BA-BI:**

The incorporation of the anti-foaming/de-foaming agent (i.e., simethicone) provided a film that not only provided a uniform film that substantially reduced or eliminated air bubbles in the film product, but also provided other benefits. The films displayed more desirable organoleptic properties. The films had an improved texture that was less "paper-like" provided a better mouth-feel to the consumer.

The compositions in Table 9 were prepared (including the addition of simethicone in inventive compositions BA-BG) and mixed under vacuum to remove air bubbles.

The resultant uncut films of inventive compositions BA-BG exhibited uniformity in content particularly with respect to the insoluble active, as well as unit doses of ¾" by 1" by 5 mils cut therefrom. The inventive compositions also were observed to have a smooth surface, absent of air bubbles. The significantly higher amounts of simethicone present in inventive compositions BF-BG also provided a very uniform film, but not significantly improved from that of inventive compositions BA-BE.

By contrast, comparative examples BH-BI were observed to have a rougher surface, exhibiting the inclusion of air bubbles in the resultant film which provided a less uniform texture and distribution of the ingredients.

**TABLE 9**

| Ingredient | BA | BB | BC | BD | BE | BF | BG | BH | BI |
|---|---|---|---|---|---|---|---|---|---|
| Hydroxypropylmethyl cellulose | 0 | 3.77 | 3.70 | 3.84 | 0 | 3.67 | 0 | 0 | 3.84 |
| Peppermint oil | 2.94 | 1.93 | 2.39 | 0 | 0 | 2.67 | 2.94 | 2.67 | 0 |
| Sweetener | 2.20 | 0.32 | 0.23 | 0 | 0.17 | 1.53 | 2.20 | 1.54 | 0 |
| Polyvinylpyrrolidone | 2.68 | 2.01 | 2.39 | 0 | 0 | 2.33 | 2.68 | 2.34 | 0 |
| Tween 80[1] | 2.24 | 1.07 | 1.48 | 1.42 | 0.55 | 1.35 | 2.24 | 0 | 1.42 |
| Simethicone[2] | 0.66 | 0.42 | 0.68 | 0.22 | 0.22 | 5.00 | 2.00 | 0 | 0 |
| Listerine[3] | 0 | 0 | 0 | 0 | 92.41 | 0 | 0 | 0 | 0 |
| Methylcellulose | 4.03 | 0 | 0 | 0 | 0 | 0 | 4.03 | 0 | 0 |
| Cornstarch[4] | 2.68 | 0 | 0 | 0 | 0 | 0 | 2.68 | 0 | 0 |
| Water | 73.53 | 90.47 | 89.14 | 92.22 | 0 | 83.45 | 72.19 | 93.46 | 92.44 |
| Loratadine[5] | 4.29 | 0 | 0 | 2.31 | 0 | 0 | 4.29 | 0 | 2.31 |
| Pullulan[6] | 0 | 0 | 0 | 0 | 6.65 | 0 | 0 | 0 | 0 |
| Calcium Carbonate | 1.43 | 0 | 0 | 0 | 0 | 0 | 1.43 | 0 | 0 |
| Xanthan Gum | 0.30 | 0 | 0 | 0 | 0 | 0 | 0.30 | 0 | 0 |
| Propylene Glycol | 3.02 | 0 | 0 | 0 | 0 | 0 | 3.02 | 0 | 0 |

[1] Available from ICI Americas
[2] Available from OSI
[3] Available from Pfizer, Inc. including thymol (0.064%), eucalyptol (0.092%), methyl salicylate (0.060%), menthol (0.042%), water (up to 72.8%), alcohol (26.9%), benzoic acid, poloxamer 407, sodium benzoate, and caramel color
[4] Available from Grain Processing Corporation as Pure Cote B792
[5] Available from Schering Corporation as Claritin
[6] Available from Hayashibara Biochemical Laboratories, Inc., Japan

## Examples CA-CC:

The following examples of the present invention describe films and film-forming compositions that use an ethoxylated caster oil as a surfactant, or alternatively are free of surfactants, plasticizers and/or polyalcohols. Desirably, the films or film-forming compositions of the present invention are essentially free of surfactants. Moreover, the films or film-forming compositions of the present invention are desirably formulated to be essentially free of surfactants. Furthermore, the films or film-forming compositions of the present invention are desirably formulated to be essentially free of plasticizers. Still furthermore, the films or film-forming compositions of the present invention are desirably formulated to be essentially free of polyalcohols. Moreover, the films or film-forming compositions of the present invention are desirably formulated to be essentially free of surfactants and plasticizers. Furthermore, the films or film-forming compositions of the present invention are desirably formulated to be essentially free of surfactants, plasticizers and polyalcohols.

TABLE 10

| Ingredient | (parts by wt.) CA |
|---|---|
| POLYMERS: | |
| Hydroxypropylmethyl cellulose | 15.6 |
| Cornstarch[1] | 10.41 |
| Polyvinylpyrrolidone | 10.41 |
| Xanthan Gum | 1.14 |
| | |
| SURFACTANT[2]: | 2.0 |
| | |
| PLASTICIZER[3]: | 11.67 |
| | |
| ANTI-FOAM AGENT[4] | 2.44 |
| | |
| OTHER | |
| Spearmint Flavor | 10.43 |
| Loratadine (drug) | 16.62 |
| Calcium Carbonate | 5.54 |
| Sweetener | 9.36 |
| [1] Available from Grain Processing Corporation as Pure Cote B792  [2] Ethoxylated caster oil, Cremophor® EL available from BASF  [3] Propylene Glycol  [4] Silicone Emulsion | |

The above ingredients were added at 30% to 70% water and stirred until polymers were fully hydrated which took 45 min. The mix was then put under vacuum to eliminate entrapped air. Vacuum was added in a steady manner starting at 500 mm and progressing up to 760 mm over 45 min.

After release of the vacuum, 6 grams of the liquid was added to a coating paper using a 200 micron spiral wound rod and a K Control Coater Model 101 (RK Print Coat Inst. Ltd.). The paper substrate onto which the coating was added was a silicone coated paper. The coated paper was then dried at 90°C until about 5% moisture remained. The formula coated and dried to a film thickness of approx. 60 microns and quickly dissolved in the mouth.

TABLE 11

| Ingredient | (parts by wt.) CB |
|---|---|
| POLYMERS: | |
| Hydroxypropylmethyl cellulose | 15.6 |
| Cornstarch[1] | 10.41 |
| Polyvinylpyrrolidone | 10.41 |
| | |
| PLASTICIZER/SOLVENT[2]: | 22.1 |
| | |
| ANTI-FOAM AGENT[3] | 2.44 |

(continued)

| Ingredient | (parts by wt.) CB |
|---|---|
| POLYMERS: | |
| | |
| OTHER | |
| Raspberry Flavor | 0.3 |
| Calcium Carbonate[4] | 30.38 |
| Sweetener | 8.36 |
| [1] Available from Grain Processing Corporation as Pure Cote B792<br>[2] Propylene Glycol<br>[3] Polydimethyl Siloxane Emulsion<br>[4] Functioned to mimic drug loading | |

The above ingredients were added to water at 40% until a homogeneous suspension was made. Vacuum was added over 20 min. starting at 500 mm Hg. and ending at 660 mm Hg. until all air was removed from suspension. Film was made as described in prior experiments. The liquid coated the silicone release substrate and dried to a uniform flexible film. The film passed the 180° bend test without cracking and dissolved in the mouth.

**TABLE 12**

| Ingredient | (parts by wt.) CC |
|---|---|
| POLYMERS: | |
| Hydroxypropylmethyl cellulose | 7.8 |
| Hydroxypropyl cellulose | 7.8 |
| | |
| ANTI-FOAM AGENT[1] | 0.75 |
| | |
| OTHER | |
| Peppermint & Bittermint Flavor | 2.25 |
| Tastemasking Flavor[2] | 0.3 |
| Calcium Carbonate[3] | 15.2 |
| Sweeteners | 0.9 |
| [1] Polydimethyl Siloxane Emulsion<br>[2] Prosweet from Virginia Dave<br>[3] Functioned to mimic drug loading | |

The above ingredients were added at 30% to 70% water and stirred until polymers were fully hydrated which took 20 min. The mix was then put under vacuum to eliminate entrapped air. Vacuum was added in a steady manner up to 760 mm over 35 min.

After release of the vacuum, the liquid was added to a coating paper using a 350 micron smooth bar and a K Control Coater Model 101 (RK Print Coat Inst. Ltd.). The paper substrate onto which the coating was added was a silicone coated paper. The coated paper was then dried at 90°C until about 4% moisture remained. The formula coated and dried to a film. The film had an acceptable taste and quickly dissolved in the mouth. The taste-masking flavor is an ingredient that affects the taste receptors to mask the receptors from registering a different, typical undesirable, taste. The film passed the 180° bend test without cracking and dissolved in the mouth.

Example CD:

The following example of the present invention describe films and film-forming compositions that use a taste-masked, pharmaceutically active agent which also contains flavors and taste-masking aids. A taste-masking flavor is an ingredients that effects taste receptors to mask the receptors from registering a different, typically undesirable, taste.

**TABLE 13**

| Ingredient | (grams) CD |
|---|---|
| Hydroxypropylmethyl cellulose | 4.26 |
| Hydroxypropyl cellulose | 1.42 |
| Precipitate calcium Carbonate | 1.22 |
| Sweetener[1] | 0.6 |
| Taste-Masking flavor[2] | 0.08 |
| Taste-masked Acetaminophen[3] | 5.86 |
| Cinnamon Flavor | 0.9 |
| Spearmint Flavor | 0.43 |
| polydimethylsiloxane emulsion | 0.23 |
| [1] Sucralose, available from McNeil Nutritionals [2] Magna Sweet, available from Mafco Worldwide Corp. [3] Gutte Enteric, coated acetaminophen, Gatte, LLC | |

The above ingredients, except for the pharmaceutically active agent and flavors, were added at 35 grams water and stirred until polymers were fully hydrated which took about 20 min. Food coloring (7 drops of red food coloring and 1 drop of yellow fool coloring) was also added. The mix was then put under vacuum to eliminate entrapped air. Vacuum was added in a steady manner starting at 500 mm and progressing up to 760 mm over about 10 to 20 minutes. The taste-masked Acetaminophen was added to the mix in about 4 minutes was stirring under vacuum. The flavors were then added to the mix in about 4 minutes was stirring under vacuum.

After release of the vacuum, the liquid solution was added to a coating paper using a 350 micron smooth bar. The paper substrate onto which the coating was added was a silicone coated paper. The coated paper was then dried at 90°C for about 11 minutes until about 3% moisture remained.

The formula coated and dried to a film. The film had an acceptable taste and moderately quickly dissolved in the mouth. The film did not curl on standing. The film passed the 180° bend test without cracking and dissolved in the mouth.

**Claims**

1. A film product formed by the steps of:

   (a) combining a water soluble polymer and water to form a material with a non-self-aggregating uniform heterogeneity;
   (b) forming said material into a film having a top side and a bottom side; and
   (c) drying said film from the bottom of said film to the top of said film at a temperature of 100 °C or less by applying heat to the bottom side wherein the drying occurs within the first 0.5 to 4.0 minutes.

2. The film product of claim 1, wherein said film is dried by applying hot air currents to the bottom of said film with substantially no top air flow.

3. The film product of claim 1 or 2, wherein the water content of said dried film has an upper level of about 10 % by weight.

4. The film product of any one of claims 1 to 3, wherein water added in step (a) has a weight percent of at least about 30 %.

5. The film product of any one of claims 1 to 4, wherein said drying of said film reduces the weight percent of water to about 8 % or less.

6. The film product of claim 5, wherein said drying of said film reduces the weight percent of water to about 6 % or less.

7. The film product of any one of claims 1 to 6, wherein said polymer is a cellulose derivative.

8. The film product of any one of claims 1 to 6, wherein said water soluble polymer is a member selected from the group consisting of hydroxypropylmethyl cellulose, hydroxyethyl cellulose, hydroxypropyl cellulose, polyvinylpyrrolidone, carboxymethyl cellulose, polyvinyl alcohol, sodium aginate, polyethylene glycol, xanthan gum, tragancanth gum, guar gum, acacia gum, arabic gum, polyacrylic acid, methylmethacrylate copolymer, carboxyvinyl copolymers, starch and combinations thereof.

9. The film product of any one of claims 1 to 8, wherein said film product has a thickness of greater than about 2.54 $\mu$m (0.1 mils).

10. The film product of any one of claims 1 to 8, wherein said film product has a thickness of about 254 $\mu$m (10 mils) or fewer.

11. The film product of any one of claims 1 to 10, wherein said film product has a substantially uniform thickness.

12. The film product of any one of claims 1 to 11, further comprising an active component.

13. The film product of claim 12, wherein said active component is a member selected from the group consisting of medicaments, flavors, fragrances, enzymes, preservatives, sweetening agents, colorants, spices, vitamins, and combinations thereof.

14. The film product of claim 12 or 13, wherein said film product is divided into dosage forms of substantially equal dimensions.

15. The film product of claim 14, wherein each of said dosage forms contains a substantially equal amount of said active component.

16. The film product of claim 14, wherein said dosage forms contain an amount of said active component that varies about 10 % or less among said dosage forms.

17. The film product of any one of claims 1 to 16, further including an antifoaming agent, preferably simethicone.

18. The film product of any one of claims 1 to 17, wherein the film product is essentially free of a surfactant.

19. The film product of any one of claims 1 to 18, wherein the film product is essentially free of a plasticizer.

20. The film product of any one of claims 1 to 19, wherein the film product is essentially free of a polyalcohol.

21. The film product of any one of claims 1 to 20, wherein said film is ingestible.

22. The film product of any one of claims 1 to 21, wherein said film is flexible when dried.

23. The film product of any one of claims 1 to 22, wherein said film is self-supporting.

24. The film product of any one of claims 1 to 23, wherein said film is formed by a further step (d) comprising employing other methods of heating.

25. A process for making a film product as claimed in any one of claims 1 to 24, having a substantially uniform distribution of components, comprising:

    (a) combining a water soluble polymer component and water to form a matrix with a uniform distribution of said components;

(b) forming a film from said matrix;

(c) providing a surface having top and bottom sides;

(d) feeding said film onto said top side of said surface; and

(e) drying said film from the bottom of said film to the top of said film at a temperature of 100 °C or less by applying heat to the bottom side wherein the drying occurs within the first 0.5 to 4.0 minutes.

26. The process of claim 25, wherein said film is dried by applying hot air currents to the bottom of said film with substantially no top air flow.

27. The process of claim 25 or 26, wherein the water content of said dried film has an upper level of about 10 % by weight.

28. The process of any one of claims 25 to 27, further comprising the step of adding an active component to said matrix of step (a).

29. The process of any one of claims 25 to 28, wherein said film is formed from said matrix by reverse roll coating.

30. The process of any one of claims 25 to 29, further comprising the step (f) of employing other methods of heating.

31. A dosage form for the administration of an active component comprising:

(a) a first layer comprising a film product as claimed in any one of claims 1 to 24, formed by the steps of:

(i) combining a water soluble polymer, an active component, and water to form a material with a non-self-aggregating uniform heterogeneity;

(ii) forming said material into a film; and

(iii) drying said film from the bottom of said film to the top of said film at a temperature of 100 °C or less by applying heat to the bottom side wherein the drying occurs within the first 0.5 to 4.0 minutes; and

(b) a substantially non-water soluble second layer.

32. The dosage form of claim 31, wherein said film is dried by applying hot air currents to the bottom of said film with substantially no top air flow.

33. The dosage form of claim 31, wherein the water content of said dried film has an upper level of about 10 % by weight.

34. The dosage form of any one of claims 31 to 33, wherein said first layer is removable from said second layer.

35. The dosage form of any one of claims 31 to 33, wherein said film may be applied to the tongue of a mammal.

36. The dosage form of any one of claims 31 to 33, wherein said film has a shape comprising first and second opposing bases wherein first base is longer than said second base.

37. The dosage form of any one of claims 31 to 33, wherein said film has a shape selected from the group consisting of trapezoid and triangle.

38. The dosage form of any one of claims 31 to 33, wherein said film adheres to an oral cavity.

39. The dosage form of any one of claims 31 to 33, wherein said film includes an adhesive to adhere said film to an oral cavity.

40. The dosage form of any one of claims 31 to 39, comprising a film having a uniformly dispersed composition comprising a water-soluble polymer, a pharmaceutical and/or cosmetic active component and a water, said film being formed by depositing a wet film of said composition onto a substrate surface and controllably drying the wet film from the side contacting the substrate to prevent self-aggregation and achieve compositional uniformity.

41. The dosage form of any one of claims 31 to 40, comprising a polymeric film having no more than a 10 % variance of a pharmaceutical and/or cosmetic active per unit area.

**42.** The dosage form of any one of claims 31 to 41, further including simethicone.

**43.** The dosage form of any one of claims 31 to 42, wherein the film product of the first layer (a) is formed by a further step (iv) comprising employing other methods of heating.

**44.** An edible water-soluble delivery system in the form of a film composition comprising:

a water-soluble polymer comprising hydroxypropylmethyl cellulose, hydroxypropyl cellulose, and combinations thereof; and
an active component selected from the group consisting of cosmetic agents, pharmaceutical agents, bioactive agents and combinations thereof;
wherein the delivery system is essentially free of plasticizers, surfactants and polyalcohols,

wherein said delivery system is formed by combining said water soluble polymer and water to form a material with a non-self-aggregating uniform heterogeneity, forming said material into a film and drying said film from the bottom of said film to the top of said film at a temperature of 100 °C or less by applying heat to the bottom side wherein the drying occurs within the first 0.5 to 4.0 minutes.

**45.** The edible water-soluble delivery system of claim 44, wherein said film is dried by applying hot air currents to the bottom of said film with substantially no top air flow.

**46.** The edible water-soluble delivery system of claim 44 or 45, wherein the water content of said dried film has an upper level of about 10 % by weight.

**47.** The edible water-soluble delivery system of any one of claims 44 to 46, wherein said active component is present in amounts of up to about 0.1 % to about 60 % by weight of the total delivery system.

**48.** The edible water-soluble delivery system of claim 44 to 47, wherein drying said film further comprises a step employing other methods of heating.

**49.** A pharmaceutical composition in the form of a film for enteral or topical administration, comprising a composition having a uniformly distributed combination of a water soluble polymer, water, and a pharmaceutical active component, said composition in its dried film form maintaining the uniform distribution of components through the application of controlled bottom drying of the film,
wherein said controlled bottom drying comprises drying said film from the bottom of said film to the top of said film at a temperature of 100 °C or less by applying heat to the bottom side wherein the drying occurs within the first 0.5 to 4.0 minutes.

**50.** The pharmaceutical composition of claim 49, wherein said film is dried by applying hot air currents to the bottom of said film with substantially no top air flow.

**51.** The pharmaceutical composition of claim 49 or 50, wherein the water content of said dried film has an upper level of about 10 % by weight.

**52.** The pharmaceutical composition of any one of claims 49 to 51 in unit dosage form sealed in a pouch.

**53.** The pharmaceutical composition of any one of claims 49 to 52, further including simethicone.

**54.** The pharmaceutical composition of any one of claims 49 to 53, in the form of a pharmaceutical dispenser comprising individual unit dosage forms of the pharmaceutical composition.

**55.** The pharmaceutical composition of claim 54, wherein said individual unit dosage forms are in a roll or stacked in the dispenser.

**56.** The pharmaceutical composition of any of claims 49 to 55, wherein drying said film further comprises a step employing other methods of heating.

**Patentansprüche**

1.  Filmprodukt, das durch folgende Schritte gebildet wird:

    (a) Kombinieren eines wasserlöslichen Polymers mit Wasser unter Bildung eines Materials mit nicht selbstaggregierender einheitlicher Heterogenität,
    (b) Formen des Materials zu einem Film mit einer Oberseite und einer Unterseite; und
    (c) Trocknen des Films von unten nach oben bei einer Temperatur von 100 °C oder weniger, indem der Unterseite Wärme zugeführt wird, wobei das Trocknen innerhalb der ersten 0,5 bis 4,0 Minuten stattfindet.

2.  Filmprodukt nach Anspruch 1, wobei der Film durch Zufuhr warmer Luftströme auf die Unterseite des Films mit im Wesentlichen keinem Luftstrom auf der Oberseite getrocknet wird.

3.  Filmprodukt nach Anspruch 1 oder 2, wobei der Wassergehalt des getrockneten Films einen oberen Wert von ungefähr 10 Gewichts-% aufweist.

4.  Filmprodukt nach einem der Ansprüche 1 bis 3, wobei das in Schritt (a) zugegebene Wasser einen Gewichtsanteil von wenigstens ungefähr 30 % aufweist.

5.  Filmprodukt nach einem der Ansprüche 1 bis 4, wobei das Trocknen des Films den Gewichtsanteil von Wasser auf ungefähr 8 % oder weniger verringert.

6.  Filmprodukt nach Anspruch 5, wobei das Trocknen des Films den Gewichtsanteil von Wasser auf ungefähr 6 % oder weniger verringert.

7.  Filmprodukt nach einem der Ansprüche 1 bis 6, wobei das Polymer ein Cellulosederivat ist.

8.  Filmprodukt nach einem der Ansprüche 1 bis 6, wobei das wasserlösliche Polymer ein Mitglied ausgewählt aus der Gruppe bestehend aus Hydroxypropylmethylcellulose, Hydroxyethylcellulose, Hydroxypropylcellulose, Polyvinylpyrrolidon, Carboxylmethylcellulose, Polyvinylalkohol, Natriumaginat, Polyethylenglycol, Xanthangummi, Tragantgummi, Guar Gum, Akaziengummi, Gummi arabicum, Polyacrylsäure, Methylmethacrylatcopolymer, Carboxyvinylcopolymere, Stärke und Kombinationen davon ist.

9.  Filmprodukt nach einem der Ansprüche 1 bis 8, wobei das Filmprodukt eine Dicke von mehr als ungefähr 2,54 $\mu$m (0,1 Mil) aufweist.

10. Filmprodukt nach einem der Ansprüche 1 bis 8, wobei das Filmprodukt eine Dicke von ungefähr 2,54 $\mu$m (0,1 Mil) oder weniger aufweist.

11. Filmprodukt nach einem der Ansprüche 1 bis 10, wobei das Filmprodukt eine im Wesentlichen einheitliche Dicke aufweist.

12. Filmprodukt nach einem der Ansprüche 1 bis 11, das weiterhin einen wirksamen Bestandteil umfasst.

13. Filmprodukt nach Anspruch 12, wobei der wirksame Bestandteil ausgewählt ist aus der Gruppe, bestehend aus Medikamenten, Geschmacksstoffen, Duftstoffen, Enzymen, Konservierungsstoffen, Süßstoffen, Farbstoffen, Gewürzen, Vitaminen und Kombinationen davon.

14. Filmprodukt nach Anspruch 12 oder 13, wobei das Filmprodukt in Darreichungsformen aufgeteilt ist, die im Wesentlichen gleiche Dimensionen aufweisen.

15. Filmprodukt nach Anspruch 14, wobei jede der Darreichungsformen eine im Wesentlichen gleiche Menge des wirksamen Bestandteils enthält.

16. Filmprodukt nach Anspruch 14, wobei die Darreichungsformen eine Menge des wirksamen Bestandteils enthalten, die um ungefähr 10 % oder weniger in den Dosierungsformen variiert.

17. Filmprodukt nach einem der Ansprüche 1 bis 16, das weiterhin ein Antischaummittel, vorzugsweise Simethicon enthält.

**18.** Filmprodukt nach einem der Ansprüche 1 bis 17, wobei das Filmprodukt im Wesentlichen frei von einem oberflächenaktiven Mittel ist.

**19.** Filmprodukt nach einem der Ansprüche 1 bis 18, wobei das Filmprodukt im Wesentlichen frei von einem Weichmacher ist.

**20.** Filmprodukt nach einem der Ansprüche 1 bis 19, wobei das Filmprodukt im Wesentlichen frei von einem Polyalkohol ist.

**21.** Filmprodukt nach einem der Ansprüche 1 bis 20, wobei das Filmprodukt einnehmbar ist.

**22.** Filmprodukt nach einem der Ansprüche 1 bis 21, wobei das Filmprodukt nach Trocknung flexibel ist.

**23.** Filmprodukt nach einem der Ansprüche 1 bis 22, wobei das Filmprodukt im Wesentlichen selbsttragend ist.

**24.** Filmprodukt nach einem der Ansprüche 1 bis 23, wobei der Film durch einen weiteren Schritt (d), umfassend die Anwendung anderer Verfahren zur Erwärmung, gebildet wird.

**25.** Verfahren zur Herstellung eines Filmprodukts nach einem der Ansprüche 1 bis 24 mit im Wesentlichen einheitlicher Verteilung der Bestandteile, das Folgendes umfasst:

(a) Kombinieren eines wasserlöslichen Polymerbestandteils mit Wasser unter Bildung einer Matrix mit einheitlicher Verteilung der Bestandteile;
(b) Bilden eines Films aus der Matrix; und
(c) Bereitstellen einer Oberfläche mit Ober- und Unterseite;
(d) Aufbringen des Films auf der Oberseite der Fläche; und
(e) Trocknen des Films von unten nach oben bei einer Temperatur von 100 °C oder weniger, indem der Unterseite Wärme zugeführt wird, wobei das Trocknen innerhalb der ersten 0,5 bis 4,0 Minuten stattfindet.

**26.** Verfahren nach Anspruch 25, wobei der Film durch Zufuhr warmer Luftströme auf die Unterseite des Films mit im Wesentlichen keinem Luftstrom auf der Oberseite getrocknet wird.

**27.** Verfahren nach Anspruch 25 oder 26, wobei der Wassergehalt des getrockneten Films einen oberen Wert von ungefähr 10 Gewichts-% aufweist.

**28.** Verfahren nach einem der Ansprüche 25 bis 27, das weiterhin den Schritt des Zugebens eines wirksamen Bestandteils zur Matrix von Schritt (a) umfasst.

**29.** Verfahren nach einem der Ansprüche 25 bis 28, wobei der Film aus der Matrix durch Umkehr-Rollbeschichtung gebildet wird.

**30.** Verfahren nach einem der Ansprüche 25 bis 29, das weiterhin den Schritt (f) des Anwendens anderer Verfahren zur Erwärmung umfasst.

**31.** Darreichungsform zur Verabreichung eines wirksamen Bestandteils, umfassend:

(a) eine erste Schicht, umfassend ein Filmprodukt nach einem der Ansprüche 1 bis 24, das durch folgende Schritte gebildet wird:

(i) Kombinieren eines wasserlöslichen Polymers und eines wirksamen Bestandteils mit Wasser unter Bildung eines Materials mit nicht selbstaggregierender einheitlicher Heterogenität;
(ii) Formen des Materials zu einem Film; und
(iii) Trocknen des Films von unten nach oben bei einer Temperatur von 100 °C oder weniger, indem der Unterseite Wärme zugeführt wird, wobei das Trocknen innerhalb der ersten 0,5 bis 4,0 Minuten stattfindet; und

(b) eine im Wesentlichen wasserunlösliche zweite Schicht.

32. Darreichungsform nach Anspruch 31, wobei der Film durch Zufuhr warmer Luftströme auf die Unterseite des Films mit im Wesentlichen keinem Luftstrom auf der Oberseite getrocknet wird.

33. Darreichungsform nach Anspruch 31, wobei der Wassergehalt des getrockneten Films einen oberen Wert von ungefähr 10 Gewichts-% aufweist.

34. Darreichungsform nach einem der Ansprüche 31 bis 33, wobei die erste Schicht von der zweiten Schicht entfernbar ist.

35. Darreichungsform nach einem der Ansprüche 31 bis 33, wobei der Film auf die Zunge eines Säugers gelegt werden kann.

36. Darreichungsform nach einem der Ansprüche 31 bis 33, wobei der Film eine Form aufweist, die eine erste und zweite sich gegenüberliegende Grundfläche umfasst, wobei die erste Grundfläche länger als die zweite Grundfläche ist.

37. Darreichungsform nach einem der Ansprüche 31 bis 33, wobei der Film eine Form aufweist, die ausgewählt ist aus der Gruppe bestehend aus einem Trapezoid und Dreieck.

38. Darreichungsform nach einem der Ansprüche 31 bis 33, wobei der Film in einer Mundhöhle haftet.

39. Darreichungsform nach einem der Ansprüche 31 bis 33, wobei der Film einen Klebstoff zum Haften des Films an eine Mundhöhle umfasst.

40. Darreichungsform nach einem der Ansprüche 31 bis 39, umfassend einen Film mit einheitlich verteilter Zusammensetzung, umfassend ein wasserlösliches Polymer, einen pharmazeutisch und/oder kosmetisch wirksamen Bestandteil und Wasser, wobei der Film durch Absetzen eines nassen Films der Zusammensetzung auf eine Substratoberfläche und kontrollierbares Trocknen des nassen Films von der das Substrat berührenden Seite zur Vermeidung von Selbstaggregation und Erreichen der Einheitlichkeit in der Zusammensetzung gebildet wird.

41. Darreichungsform nach einem der Ansprüche 31 bis 40, umfassend einen Polymerfilm, der eine Abweichung von nicht mehr als 10 % an pharmazeutisch und/oder kosmetisch wirksamem Bestandteil pro Flächeneinheit aufweist.

42. Darreichungsform nach einem der Ansprüche 31 bis 41, die weiterhin Simethicon umfasst.

43. Darreichungsform nach einem der Ansprüche 31 bis 42, wobei das Filmprodukt der ersten Schicht (a) durch einen weiterhin Schritt (iv) gebildet wird, der das Anwenden anderer Verfahren zur Erwärmung umfasst.

44. Verzehrbares wasserlösliches Verabreichungssystem in Form einer Filmzusammensetzung, umfassend:

ein wasserlösliches Polymer, umfassend Hydroxypropylmethylcellulose, Hydroxycellulose und Kombinationen davon; und
einen wirksamen Bestandteil, der ausgewählt ist aus der Gruppe, bestehend aus kosmetischen Mitteln, pharmazeutischen Mitteln, bioaktiven Mitteln und Kombinationen davon;
wobei das Verabreichungssystem im Wesentlichen frei von Weichmachern, oberflächenaktiven Mitteln und Polyalkoholen ist,
wobei das Verabreichungssystem durch Kombinieren des wasserlöslichen Polymers mit Wasser unter Bildung eines Materials mit nicht selbstaggregierender einheitlicher Heterogenität gebildet wird, und wobei das Material zu einem Film gebildet wird und der Film vom unteren bis zum oberen Ende des Films bei einer Temperatur von 100 °C oder weniger getrocknet wird, indem der Unterseite Wärme zugeführt wird, wobei das Trocknen innerhalb der ersten 0,5 bis 4,0 Minuten stattfindet.

45. Verzehrbares wasserlösliches Verabreichungssystem nach Anspruch 44, wobei der Film durch Zufuhr warmer Luftströme auf die Unterseite des Films mit im Wesentlichen keinem Luftstrom auf der Oberseite getrocknet wird.

46. Verzehrbares wasserlösliches Verabreichungssystem nach Anspruch 44 oder 45, wobei der Wassergehalt des getrockneten Films einen oberen Wert von ungefähr 10 Gewichts-% aufweist.

47. Verzehrbares wasserlösliches Verabreichungssystem nach Anspruch 44 bis 46, wobei der wirksame Bestandteil in Mengen von bis zu ungefähr 0,1 Gewichts-% bis ungefähr 60 Gewichts-% bezogen auf das gesamte Abgabesystem vorhanden ist.

48. Verzehrbares wasserlösliches Verabreichungssystem nach Anspruch 44 bis 47, wobei das Trocknen des Films weiterhin einen Schritt des Anwendens anderer Verfahren zur Erwärmung umfasst.

49. Pharmazeutische Zusammensetzung in Form eines Films zur enteralen oder topischen Verabreichung, umfassend eine Zusammensetzung mit einer einheitlich verteilten Kombination eines wasserlöslichen Polymers, Wasser und einem pharmazeutisch wirksamen Bestandteil, wobei die Zusammensetzung in Form ihres getrockneten Films die einheitliche Verteilung der Bestandteile beibehält, wobei das kontrollierte Trocknen der Unterseite das Trocknen des Films von unten nach oben bei einer Temperatur von 100 °C oder weniger umfasst, indem der Unterseite Wärme zugeführt wird und wobei das Trocknen innerhalb der ersten 0,5 bis 4,0 Minuten stattfindet.

50. Pharmazeutische Zusammensetzung nach Anspruch 49, wobei der durch Zufuhr warmer Luftströme auf die Unterseite des Films mit im Wesentlichen keinem Luftstrom auf der Oberseite getrocknet wird.

51. Pharmazeutische Zusammensetzung nach Anspruch 49 oder 50, wobei der Wassergehalt des getrockneten Films einen oberen Wert von ungefähr 10 Gewichts-% aufweist.

52. Pharmazeutische Zusammensetzung nach einem der Ansprüche 49 bis 51 die in einer Einheitsdarreichungsform in einem Beutel eingeschlossen ist.

53. Pharmazeutische Zusammensetzung nach einem der Ansprüche 49 bis 52, die weiterhin Simethicon umfasst.

54. Pharmazeutische Zusammensetzung nach einem der Ansprüche 49 bis 53, in Form eines pharmazeutischen Spenders, umfassend einzelne Einheitsdarreichungsformen der pharmazeutischen Zusammensetzung.

55. Pharmazeutische Zusammensetzung nach Anspruch 54, wobei die einzelnen Einheitsdarreichungsformen sich in einer Rolle oder gestapelt in einem Spender befinden.

56. Pharmazeutische Zusammensetzung nach einem der Ansprüche 49 bis 55, wobei das Trocknen des Films weiterhin das Anwenden anderer Verfahren zur Erwärmung umfasst.


**Revendications**

1. Produit en film formé par les étapes consistant à :

   (a) combiner un polymère soluble dans l'eau et de l'eau pour former un matériau avec une hétérogénéité uniforme sans auto-agrégation ;
   (b) mettre ledit matériau sous la forme d'un film ayant un côté de dessus et un côté de dessous ; et
   (c) sécher ledit film depuis le dessous dudit film vers le dessus dudit film à une température de 100°C ou moins par application de chaleur au côté de dessous, le séchage se déroulant dans les premières 0,5 à 4,0 minutes.

2. Produit en film selon la revendication 1, dans lequel ledit film est séché par application de courants d'air chaud au dessous dudit film avec pratiquement pas de courant d'air de dessus.

3. Produit en film selon la revendication 1 ou 2, dans lequel la teneur en eau dudit film séché a un niveau supérieur d'environ 10 % en poids.

4. Produit en film selon l'une quelconque des revendications 1 à 3, dans lequel l'eau ajoutée dans l'étape (a) a un pourcentage en poids d'au moins environ 30 %.

5. Produit en film selon l'une quelconque des revendications 1 à 4, dans lequel ledit séchage dudit film réduit le pourcentage en poids d'eau à environ 8 % ou moins.

**6.** Produit en film selon la revendication 5, dans lequel ledit séchage dudit film réduit le pourcentage en poids d'eau à environ 6 % en poids ou moins.

**7.** Produit en film selon l'une quelconque des revendications 1 à 6, dans lequel ledit polymère est un dérivé de cellulose.

**8.** Produit en film selon l'une quelconque des revendications 1 à 6, dans lequel ledit polymère soluble dans l'eau est un membre choisi dans le groupe constitué par l'hydroxypropylméthylcellulose, l'hydroxyéthyl-cellulose, l'hydroxypropylcellulose, la polyvinyl-pyrrolidone, la carboxyméthylcellulose, le poly(alcool vinylique), l'alginate de sodium, le polyéthylèneglycol, la gomme xanthane, la gomme adragante, la gomme de guar, la gomme d'acacia, la gomme arabique, un poly(acide acrylique), un copolymère de méthacrylate de méthyle, les copolymères carboxyvinyliques, l'amidon et leurs combinaisons.

**9.** Produit en film selon l'une quelconque des revendications 1 à 8, lequel produit en film a une épaisseur supérieure à environ 2,54 $\mu$m (0,1 mil).

**10.** Produit en film selon l'une quelconque des revendications 1 à 8, lequel produit en film a une épaisseur d'environ 254 $\mu$m (10 mils) ou moins.

**11.** Produit en film selon l'une quelconque des revendications 1 à 10, lequel produit en film a une épaisseur sensiblement uniforme.

**12.** Produit en film selon l'une quelconque des revendications 1 à 11, comprenant en outre un composant actif.

**13.** Produit en film selon la revendication 12, dans lequel ledit composant actif est un membre choisi dans le groupe constitué par les médicaments, les arômes, les parfums, les enzymes, les conservateurs, les agents édulcorants, les colorants, les épices, les vitamines, et leurs combinaisons.

**14.** Produit en film selon la revendication 12 ou 13, lequel produit en film est divisé en formes posologiques de dimensions sensiblement identiques.

**15.** Produit en film selon la revendication 14, dans lequel chacune desdites formes posologiques contient une quantité sensiblement identique dudit composant actif.

**16.** Produit en film selon la revendication 14, dans lequel lesdites formes posologiques contiennent une quantité dudit composant actif qui varie d'environ 10 % ou moins parmi lesdites formes posologiques.

**17.** Produit en film selon l'une quelconque des revendications 1 à 16, contenant en outre un agent anti-mousse, de préférence du siméthicone.

**18.** Produit en film selon l'une quelconque des revendications 1 à 17, lequel produit en film est pratiquement exempt de tensioactif.

**19.** Produit en film selon l'une quelconque des revendications 1 à 18, lequel produit en film est pratiquement exempt de plastifiant.

**20.** Produit en film selon l'une quelconque des revendications 1 à 19, lequel produit en film est pratiquement exempt de polyol.

**21.** Produit en film selon l'une quelconque des revendications 1 à 20, dans lequel ledit film peut être ingéré.

**22.** Produit en film selon l'une quelconque des revendications 1 à 21, dans lequel ledit film est flexible quand il est séché.

**23.** Produit en film selon l'une quelconque des revendications 1 à 22, dans lequel ledit film est autoportant.

**24.** Produit en film selon l'une quelconque des revendications 1 à 23, dans lequel ledit film est formé par une étape supplémentaire (d) comprenant l'emploi d'autres procédés de chauffage.

**25.** Procédé pour préparer un produit en film tel que revendiqué dans l'une quelconque des revendications 1 à 24, ayant une distribution sensiblement uniforme des composants, consistant à :

(a) combiner un composant polymère soluble dans l'eau et de l'eau pour former une matrice avec une distribution uniforme desdits composants ;
(b) former un film à partir de ladite matrice ;
(c) disposer d'une surface ayant des côtés de dessus et de dessous ;
(d) placer ledit film sur ledit côté de dessus de ladite surface ; et
(e) sécher ledit film depuis le dessous dudit film vers le dessus dudit film à une température de 100°C ou moins par application de chaleur au côté de dessous, le séchage se déroulant dans les premières 0,5 à 4,0 minutes.

26. Procédé selon la revendication 25, dans lequel ledit film est séché par application de courants d'air chaud au dessous dudit film avec pratiquement pas de courant d'air de dessus.

27. Procédé selon la revendication 25 ou 26, dans lequel la teneur en eau dudit film séché a un niveau supérieur d'environ 10 % en poids.

28. Procédé selon l'une quelconque des revendications 25 à 27, comprenant en outre l'étape consistant à ajouter un composant actif à ladite matrice de l'étape (a).

29. Procédé selon l'une quelconque des revendications 25 à 28, dans lequel ledit film est formé à partir de ladite matrice par enduction par rouleau inversé.

30. Procédé selon l'une quelconque des revendications 25 à 29, comprenant en outre l'étape (f) consistant à employer d'autres procédés de chauffage.

31. Forme posologique pour l'administration d'un composant actif, comprenant :

(a) une première couche comprenant un produit en film tel que revendiqué dans l'une quelconque des revendications 1 à 24, formé par les étapes consistant à :

(i) combiner un polymère soluble dans l'eau, un composant actif, et de l'eau pour former un matériau avec une hétérogénéité uniforme sans auto-agrégation ;
(ii) mettre ledit matériau sous la forme d'un film ; et
(iii) sécher ledit film depuis le dessous dudit film vers le dessus dudit film à une température de 100°C ou moins par application de chaleur au côté de dessous, le séchage se déroulant dans les premières 0,5 à 4,0 minutes ; et

(b) une deuxième couche pratiquement insoluble dans l'eau.

32. Forme posologique selon la revendication 31, dans laquelle ledit film est séché par application de courants d'air chaud au dessous dudit film avec pratiquement pas de courant d'air de dessus.

33. Forme posologique selon la revendication 31, dans laquelle la teneur en eau dudit film séché a un niveau supérieur d'environ 10 % en poids.

34. Forme posologique selon l'une quelconque des revendications 31 à 33, dans laquelle ladite première couche peut être retirée de ladite deuxième couche.

35. Forme posologique selon l'une quelconque des revendications 31 à 33, dans laquelle ledit film peut être appliqué à la langue d'un mammifère.

36. Forme posologique selon l'une quelconque des revendications 31 à 33, dans laquelle ledit film a une forme comprenant des première et deuxième bases opposées, la première base étant plus longue que ladite deuxième base.

37. Forme posologique selon l'une quelconque des revendications 31 à 33, dans laquelle ledit film a une forme choisie dans le groupe constitué par un trapézoïde et un triangle.

38. Forme posologique selon l'une quelconque des revendications 31 à 33, dans laquelle ledit film adhère à la cavité orale.

**39.** Forme posologique selon l'une quelconque des revendications 31 à 33, dans laquelle ledit film contient un adhésif pour que ledit film adhère à la cavité orale.

**40.** Forme posologique selon l'une quelconque des revendications 31 à 39, comprenant un film ayant une composition uniformément dispersée comprenant un polymère soluble dans l'eau, un composant actif pharmaceutique et/ou cosmétique et de l'eau, ledit film étant formé par déposition d'un film humide de ladite composition sur une surface de substrat et séchage contrôlé du film humide depuis le côté au contact du substrat pour empêcher une auto-agrégation et obtenir une uniformité de composition.

**41.** Forme posologique selon l'une quelconque des revendications 31 à 40, comprenant un film polymère dont la variance d'agent actif pharmaceutique et/ou cosmétique par unité de surface est d'au plus 10 %.

**42.** Forme posologique selon l'une quelconque des revendications 31 à 41, comprenant en outre du siméthicone.

**43.** Forme posologique selon l'une quelconque des revendications 31 à 42, dans laquelle le produit en film de la première couche (a) est formé par une étape supplémentaire (iv) comprenant l'emploi d'autres procédés de chauffage.

**44.** Système de délivrance soluble dans l'eau et comestible sous la forme d'une composition en film comprenant :

un polymère soluble dans l'eau comprenant de l'hydroxypropylméthylcellulose, de l'hydroxypropyl-cellulose, et leurs combinaisons ; et
un composant actif choisi dans le groupe constitué par les agents cosmétiques, les agents pharmaceutiques, les agents bioactifs et leurs combinaisons ;
lequel système de délivrance est pratiquement exempt de plastifiants, tensioactifs et polyols,
lequel système de délivrance est formé par combinaison dudit polymère soluble dans l'eau et d'eau pour former un matériau avec une hétérogénéité uniforme sans auto-agrégation, mettre le matériau sous la forme d'un film et sécher ledit film depuis le dessous dudit film vers le dessus dudit film à une température de 100°C ou moins par application de chaleur au côté de dessous, le séchage se déroulant dans les premières 0,5 à 4,0 minutes.

**45.** Système de délivrance soluble dans l'eau et comestible selon la revendication 44, dans lequel ledit film est séché par application de courants d'air chaud au dessous dudit film avec pratiquement pas de courant d'air de dessus.

**46.** Système de délivrance soluble dans l'eau et comestible selon la revendication 44 ou 45, dans lequel la teneur en eau dudit film séché a un niveau supérieur d'environ 10 % en poids.

**47.** Système de délivrance soluble dans l'eau et comestible selon l'une quelconque des revendications 44 à 46, dans lequel ledit composant actif est présent en des quantités allant d'environ 0,1 % à environ 60 % en poids du système de délivrance total.

**48.** Système de délivrance soluble dans l'eau et comestible selon les revendications 44 à 47, dans lequel le séchage dudit film comprend en outre une étape employant d'autres procédés de chauffage.

**49.** Composition pharmaceutique sous la forme d'un film pour une administration par voie entérale ou topique, comprenant une composition ayant une combinaison uniformément distribuée d'un polymère soluble dans l'eau, d'eau, et d'un composant pharmaceutique actif, ladite composition, sous sa forme de film séché, conservant une distribution uniforme des composants par l'application d'un séchage contrôlé du film par le dessous,
dans laquelle ledit séchage contrôlé par le dessous comprend le séchage dudit film depuis le dessous dudit film vers le dessus dudit film à une température de 100°C ou moins par application de chaleur au côté de dessous, le séchage se déroulant dans les premières 0,5 à 4,0 minutes.

**50.** Composition pharmaceutique selon la revendication 49, dans laquelle ledit film est séché par application de courants d'air chaud au dessous dudit film avec pratiquement pas de courant d'air de dessus.

**51.** Composition pharmaceutique selon la revendication 49 ou 50, dans laquelle la teneur en eau dudit film séché a un niveau supérieur d'environ 10 % en poids.

**52.** Composition pharmaceutique selon l'une quelconque des revendications 49 à 51, sous forme posologique unitaire scellée dans une poche.

**53.** Composition pharmaceutique selon l'une quelconque des revendications 49 à 52, contenant en outre du siméthicone.

**54.** Composition pharmaceutique selon l'une quelconque des revendications 49 à 53, sous la forme d'un distributeur pharmaceutique comprenant des formes posologiques unitaires individuelles de la composition pharmaceutique.

**55.** Composition pharmaceutique selon la revendication 54, dans laquelle lesdites formes posologiques unitaires individuelles sont dans un rouleau ou sont empilées dans le distributeur.

**56.** Composition pharmaceutique selon l'une quelconque des revendications 49 à 55, dans laquelle le séchage dudit film comprend en outre une étape employant d'autres procédés de chauffage.

*10*
*14*
*12*
*14*

FIG. 1

*10*
*10'*
*16*

FIG. 2

*10*
*16*
*10'*

FIG. 3

*10*
*18*

FIG. 4

*10'*
*16*
*10*

FIG. 5

*FIG. 6*

**FIG. 7**

**EP 1 463 491 B1**

**Patent documents cited in the description**

- US 4136145 A, Fuchs
- US 4849246 A, Schmidt
- US 5629003 A, Horstmann
- US 5948430 A, Zerbe
- WO 0191721 A
- US 6284264 B
- US 5393528 A
- EP 1110546 A
- EP 514691 A
- US 4925670 A
- US 4631837 A, Magoon